(19) Europäisches Patentamt / European Patent Office / Office européen des brevets

(11) **EP 4 636 402 A1**

(12) **EUROPEAN PATENT APPLICATION**
published in accordance with Art. 153(4) EPC

(43) Date of publication:
**22.10.2025 Bulletin 2025/43**

(21) Application number: **23903922.5**

(22) Date of filing: **11.12.2023**

(51) International Patent Classification (IPC):
***G01N 33/574*** (2006.01) ***C12Q 1/6886*** (2018.01)

(52) Cooperative Patent Classification (CPC):
**C12Q 1/6886; G01N 33/543; G01N 33/574;
G01N 33/58**

(86) International application number:
**PCT/KR2023/020308**

(87) International publication number:
**WO 2024/128719 (20.06.2024 Gazette 2024/25)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **12.12.2022 KR 20220172870**

(71) Applicants:
• **Institute for Basic Science
Yuseong-gu
Daejeon 34126 (KR)**
• **Ulsan National Institute of Science and
Technology
(UNIST)
Eonyang-eup Ulju-gun
Ulsan 44919 (KR)**

(72) Inventors:
• **CHO, Yoon-Kyoung
Ulju-gun Ulsan 44920 (KR)**
• **KUMAR, Sumit
Ulju-gun Ulsan 44919 (KR)**
• **CLARISSA, Elizabeth Maria
Ulju-gun Ulsan 44919 (KR)**

(74) Representative: **Mewburn Ellis LLP
Aurora Building
Counterslip
Bristol BS1 6BX (GB)**

Remarks:
The complete document including Reference
Table(s) and the Sequence Listing(s) can be
downloaded from the EPO website

(54) **METHOD FOR DETECTING HIGH SENSITIVITY OF CANCER CELL-DERIVED EXTRACELLULAR VESICLE GENE BY USING FUSION REACTION WITH LIPOSOMES**

(57) The present invention successfully introduced a new approach to target specific EV subpopulations on the basis of charge-mediated fusion of EVs and CLIPs. By adjusting the surface charge of liposomes through the ratio of positively and negatively charged lipids, the optimal ratio that allows efficient and stable fusion with exosomes was confirmed. A method according to the present invention uses the advantages of a CLIP's high fusion rate, and rapid and broad applicability, and verified excellent sensitivity and selectivity for tumorderived EV miRNA in a lysis-free manner using dropletmicrofluidics. Particularly, the EV-CLIP method enables digital detection of EGFR L858R and T790M mutations without pretreating a sample, and thus can simplify detection processes and prevent EV loss.

EP 4 636 402 A1

FIG. 1

a

Patient plasma (20 μL)

Extracellular vesicles (EVs)

Fusion inside droplet reactor

EV-CLIP Method

Molecular beacon (MB) loaded charged-liposome (CLIP)

Digital detection of EV-RNA

*EGFR* mutation

● L858R
● T790M
● L858R + T790M

b

Aqueous phase (MB)

DOPC

Oil phase

CLIP

DOTAP

Aqueous phase (MB)

CLIP preparation

200 μm

Microfluidic hydrodynamic focusing (MHF) chip

CLIP

Tuning surface charge of CLIP

c

50 nm

CLIP

d

<0.0001
<0.0001
<0.0001
<0.0001

Zeta potential (mV)

$\chi_{DOTAP}$:$\chi_{DOPC}$ (mol %)

0:100  25:75  50:50  75:25  100:0

e

0.095
0.1806
0.1669
0.1418

Size, $D_H$ (nm)

$\chi_{DOTAP}$:$\chi_{DOPC}$ (mol %)

0:100  25:75  50:50  75:25  100:0

# EP 4 636 402 A1

## Description

### TECHNICAL FIELD

The present invention relates to a charged-liposome for detecting an extracellular vesicle (EV) and the use thereof, and more particularly, to a liposome for detecting an extracellular vesicle comprising a cationic lipid and a neutral lipid, and a composition for detecting a cancer cell-derived extracellular vesicle, a detection kit, a detection method, a composition for diagnosing cancer, a kit for diagnosing cancer, a method of providing information for diagnosing cancer, and a method of diagnosing cancer, comprising the liposome.

### BACKGROUND ART

The analysis of single extracellular vesicle (EV) has emerged as a powerful tool in the field of biomedical research, providing valuable insights into a variety of diseases and disorders (Sullivan, L. B., Nature Chemical Biology 2017, 13(9), 924-925). This allows for detailed investigation of EV heterogeneity (Tkach, M.; Thery, C., Cell 2016, 164(6), 1226-1232), identification of disease-specific biomarkers (Peinado, H., et al., Nature Medicine 2012, 18(6), 883-891), and monitoring of dynamic changes in disease progression by identifying unique nucleic acids (Cocks, A., et al., Seminars in Cancer Biology 2021, 75, 127-135), proteins (Whittle, K., et al., Critical Reviews in Oncology/Hematology 2022, 171, 103603), or other molecules (Yu, W., et al., Ann Oncol 2021, 32(4), 466-477), which are indicative of a particular disease. Therefore, investigating the contents and characteristics of single EV has the potential to reveal important information about disease processes (Marar, C., et al., Nature Immunology 2021, 22(5), 560-570). In order to detect EV-derived RNA with high sensitivity, the optimal standard quantitative reverse transcription-polymerase chain reaction (qRT-PCR) (Gandham, S., et al., Trends Biotechnol 2020, 38(10), 1066-1098) and various novel approaches have been proposed (Shao, H., et al., Chem Rev 2018, 118(4), 1917-1950). Despite the notable advantages such methods offer, most of these detection methods still present challenges that require attention, especially due to the inclusion of laborious and time-consuming steps such as EV isolation from biological samples such as plasma, EV lysis, RNA extraction, and reverse transcription amplification (Erdbrugger, U.; Lannigan, J., Cytometry Part A 2016, 89(2), 123-134). In addition, since biological samples contain a mixture of EVs derived from tumor cells and EVs derived from non-tumor cells and also since conventional methods for RNA isolation and analysis from bulk solutions are performed, distinguishing tumor cell-derived RNA signals continues to be a challenging task (Bordanaba-Florit, G., et al., Nature Protocols 2021, 16(7), 3163-3185). Consequently, further research is essential to develop more efficient and accurate strategies for detection of tumor cell-derived EV RNA.

Membrane fusion mediated by SNARE proteins and often facilitated by calcium ions ($Ca^{2+}$), is essential for diverse cellular processes including exocytosis and endocytosis, membrane remodeling, cell division, signal transduction, and intracellular transport (Koike, S.; Jahn, R., Nature Communications 2019, 10(1), 1608). Extensive research has been conducted on fusion mechanisms of phospholipid compartments (Ma, M.; Bong, D., Accounts of Chemical Research 2013, 46(12), 2988-2997) and has been applied to develop functional systems that may be applied in diagnosis and therapy (Mazur, F.; Chandrawati, R., ChemNanoMat 2021, 7(3), 223-237). There have been devised various EV membrane fusion processes such as pH-dependent (Yang, Y., et al., Advanced Materials 2017, 29(13), 1605604), polyethylene glycol-mediated (Piffoux, M., et al., ACS Nano 2018, 12(7), 6830-6842), catechol-metal supramolecular complex (Kumar, S., et al., Nature Catalysis 2021, 4(9), 763-774), freeze-thaw cycle-mediated (Cheng, L., et al., Biomaterials 2021, 275, 120964), and DNA zipper-mediated (Peruzzi, J. A., et al., Angew Chem Int Ed Engl 2019, 58(51), 18683-18690) processes. Such processes involve a variety of molecular compositions on the plasma membrane that bind or dock to the membrane, bringing them into close proximity while inducing local disturbances, thus reducing the energy barrier for fusion. Recently, many strategies have been developed to utilize fusogenic vesicles inspired by viral infection mechanisms (Gao, X., et al., Angewandte Chemie International Edition 2019, 58(26), 8719-8723) and aptamer-mediated fusion (Feng, J., et al., Analytical Chemistry 2023, 95(19), 7743-7752) for detection of EV RNAs within natural environments. However, these methods also require complex genetic manipulations or optimization of numerous aptamers, which may be technically challenging and time-consuming, limiting utility thereof in a broad range of clinical scenarios. Therefore, it is important to develop a more generalized and simplified approach for tumor-derived EV RNA detection that can be easily applied in clinical practice.

In the present invention, the inventors successfully developed a simple, efficient, surface protein-independent, charge-induced fusion method for EV/liposome fusion within a microfluidic droplet reactor, enabling for the investigation of miRNAs and mRNAs inside individual EVs. By manipulation of the ratio of positively and negatively charged lipids, the surface charge of liposomes could be finely tuned to enable efficient fusion with EVs, with precise control of fusion ranging from less than 5% to over 60%. A certain combination of charged liposomes showed the highest fusion efficiency among different charge types. Furthermore, the present invention enables high-throughput single-vesicle miRNA or mRNA profiling by sorting individual EVs in emulsion droplets and utilizing charged-liposome (CLIP) EV detection (EV-CLIP) via droplet scanning. The present inventors successfully digitally detected EGFR L858R and T790M mutations from blood plasma samples from 73 lung cancer patients (17 without mutation, 56 with mutation) and 10 healthy donors. In particular, the innovative EV-CLIP method minimized EV loss by simplifying the detection process without sample preprocessing.

The above information in this Background Art is intended only to improve the understanding of the background of the present invention and it may not include information that constitutes prior art known to one having ordinary skill in the art to which the present invention pertains.

## SUMMARY OF THE INVENTION

It is an object of the present invention to provide a charged-liposome for detecting a cancer cell-derived extracellular vesicle (EV) gene with high sensitivity and high selectivity.

It is another object of the present invention to provide a composition for detecting a cancer cell-derived extracellular vesicle comprising the liposome, a detection kit, a detection method, a composition for diagnosing cancer, a kit for diagnosing cancer, a method of providing information for diagnosing cancer, and a method of diagnosing cancer.

In order to accomplish the above objects, the present invention provides a liposome for detecting a cancer cell-derived extracellular vesicle (EV) comprising a cationic lipid and a neutral lipid, in which a cancer cell-specific molecular beacon is encapsulated within the liposome.

The present invention also provides a composition and kit for detecting a cancer cell-derived extracellular vesicle comprising the liposome for detecting an extracellular vesicle.

The present invention also provides a method of detecting a cancer cell-derived extracellular vesicle comprising fusing the liposome for detecting an extracellular vesicle with an extracellular vesicle derived from a biological sample.

The present invention also provides a composition and kit for diagnosing cancer comprising the liposome for detecting an extracellular vesicle.

The present invention also provides a method of providing information for cancer diagnosis and a method of diagnosing cancer comprising fusing the liposome for detecting an extracellular vesicle with an extracellular vesicle derived from a biological sample.

## BRIEF DESCRIPTION OF THE DRAWINGS

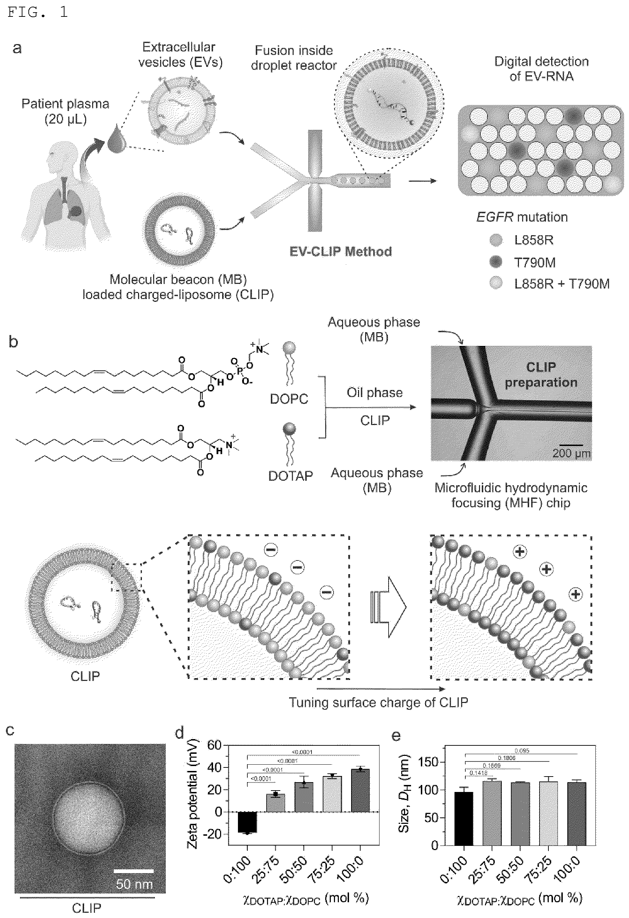

FIG. 1 shows an overview of EV RNA analysis based on EV-CLIP fusion reaction. (a) Schematic of a process for inducing a fusion reaction between CLIP loaded with a molecular beacon (MB) constructed for the RNA to be analyzed and EV in a droplet reactor and detecting specific gene mutations within EV. For example, MBs for detecting gene EGFR L858R mutation, which is associated with targeted therapy selection for lung cancer patients, are tagged with a green fluorescent molecule, and MBs for EGFR T790M, which is associated with drug resistance, are tagged with a red fluorescent molecule, and thus, when the mutant genes and MBs react within the EVs in each droplet reactor, a fluorescence signal is generated, and the number of droplets in which the fluorescence signal is detected is counted and used for digital quantitative analysis of EV-derived RNA. (b) Schematic of the CLIP (charged-liposome) preparation using microfluidic hydrodynamic focusing method. The CLIP is composed of positively charged 1,2-dioleoyl-3-trimethylammonium propane (DOTAP, red) and negatively charged 1,2-dioleoyl-sn-glycero-3-phosphocholine (DOPC, green) lipids. (c) Representative image of the CLIP taken using transmission electron microscope (TEM) (diameter: $92.5\pm5.65$ nm, n=3 with 9 data points). Scale bar: 100 nm. (d) Zeta potential of CLIPs with respect to various molar ratio compositions (%) of DOTAP and DOPC ($x_{DOTAP}$ : $x_{DOPC}$) (0% DOTAP: -18.63$\pm$0.76, 25% DOTAP: 16.17$\pm$3.05, 50% DOTAP: 26.90$\pm$5.27, 75% DOTAP: 32.20$\pm$2.26, 100% DOTAP: 38.67$\pm$2.51), in which data are presented as mean$\pm$SD and are based on three independently synthesized CLIP batches. (e) Hydrodynamic size ($D_H$) distribution of CLIPs with different ratio of DOTAP, in which size and surface charge measurements were performed using DLS (0% DOTAP: 95.82$\pm$8.87 nm, 25% DOTAP: 116.40$\pm$3.67 nm, 50% DOTAP: 113.40$\pm$1.00 nm, 75% DOTAP: 115.03$\pm$8.86 nm, 100% DOTAP: 113.7$\pm$4.3 nm). Data are presented as mean$\pm$SD and are based on three independently synthesized CLIP batches. All statistical analyses were performed by one-way ANOVA.

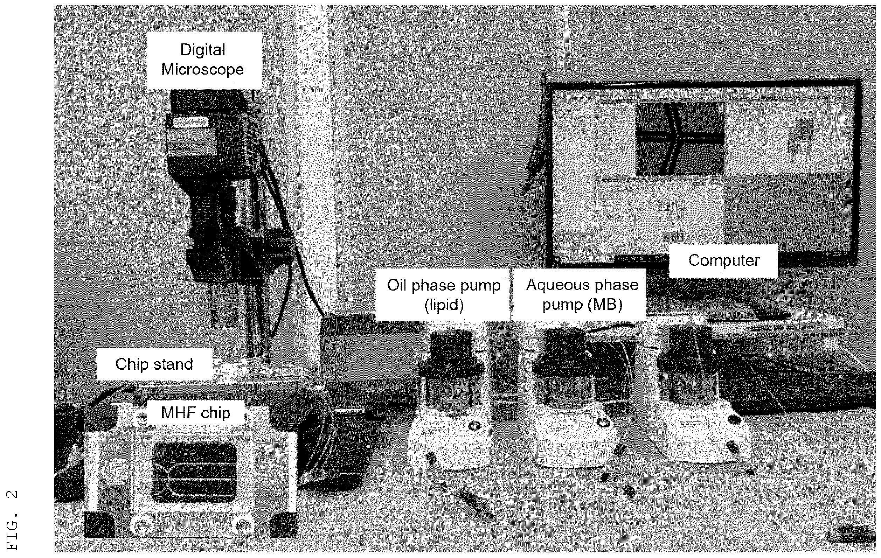

FIG. 2 shows an experimental setup for Microfluidic Hydrodynamic Focusing (MHF)-based liposome generation. The setup includes a digital microscope, a chip stand, oil and aqueous phase pumps, a computer, and a 5-input chip. Lipids dissolved in ethanol (EtOH) are loaded into the oil phase pump, PBS is loaded into the aqueous phase pump. The oil pump propels the lipids to the left side of the chip, while the aqueous pump propels the PBS to the right side of the chip. With proper flow control, the oil layer is interposed between two aqueous layers, resulting in the formation of liposomes within the 5-input chip.

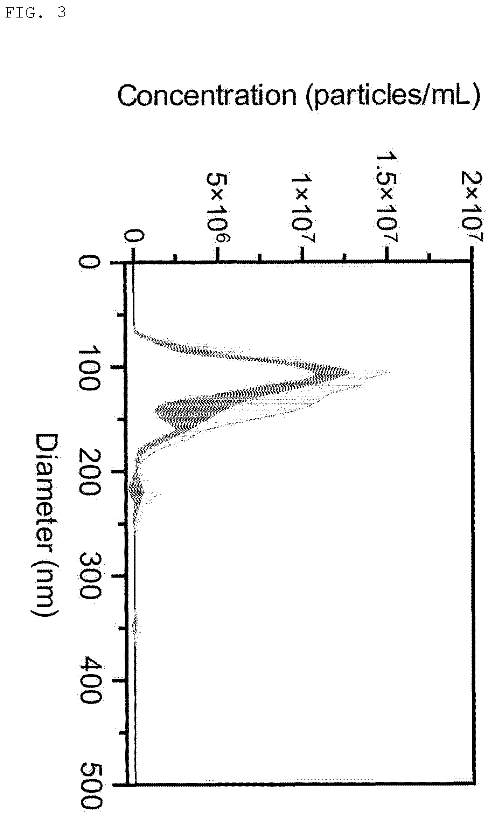

FIG. 3 shows size distribution of liposomes measured by nanoparticle tracking analysis (NTA). The liposomes showed a single peak distribution curve, indicating a homogeneous liposome population.

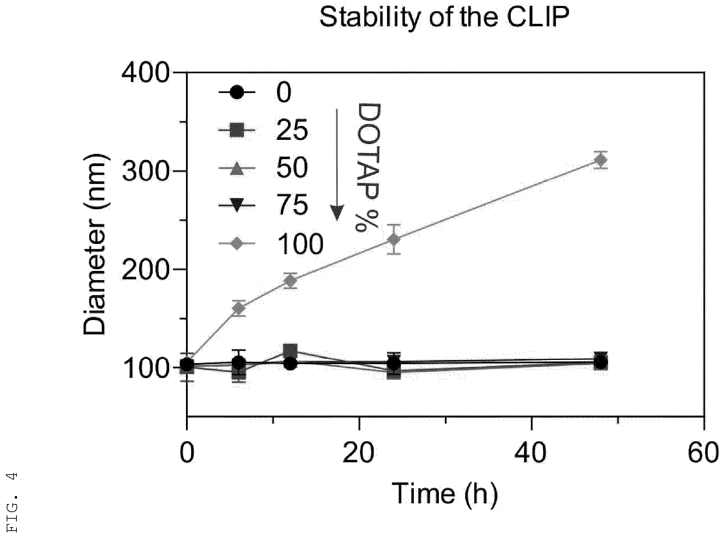

FIG. 4 shows stability of various CLIPs over time. In order to evaluate the size change of liposomes over 48 hours, the size was measured over time. The size of 0%, 25%, 50%, and 75% DOTAP remained stable over time, whereas the size of 100% DOTAP continuously increased up to 300 nm after 48 hours (6 hr: 160.53±8.15 nm, 12 hr: 188.27±7.58 nm, 24 hr: 230.37±14.96 nm, 48 hr: 311.50±8.51 nm).

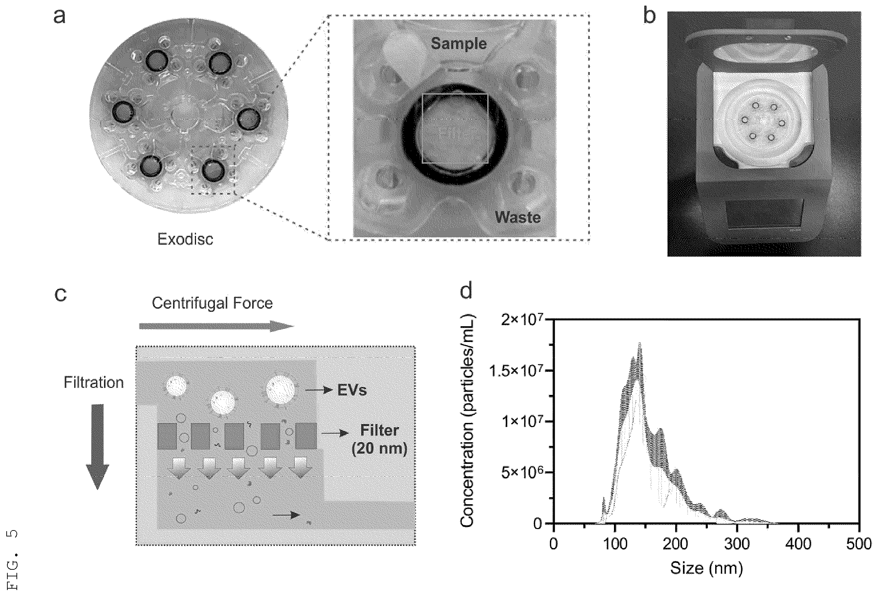

FIG. 5 shows EV isolation. (a) ExoDisc platform, a centrifugal disc equipped with anodized aluminum oxide filters with a pore diameter of 0.02 $\mu$m (Exodisc, LabSpinner). The supernatant was passed through the filter by centrifugation at 3,000 rpm (approximately 500 g), and 100 $\mu$l of concentrated EVs from the collection chamber was resuspended in 1X PBS with a dilution factor of 2. (b) Tabletop centrifuge platform for ExoDisc operation. (c) A principle of ExoDisc operation. (d) The EVs were characterized by nanoparticle tracking analysis (NTA).

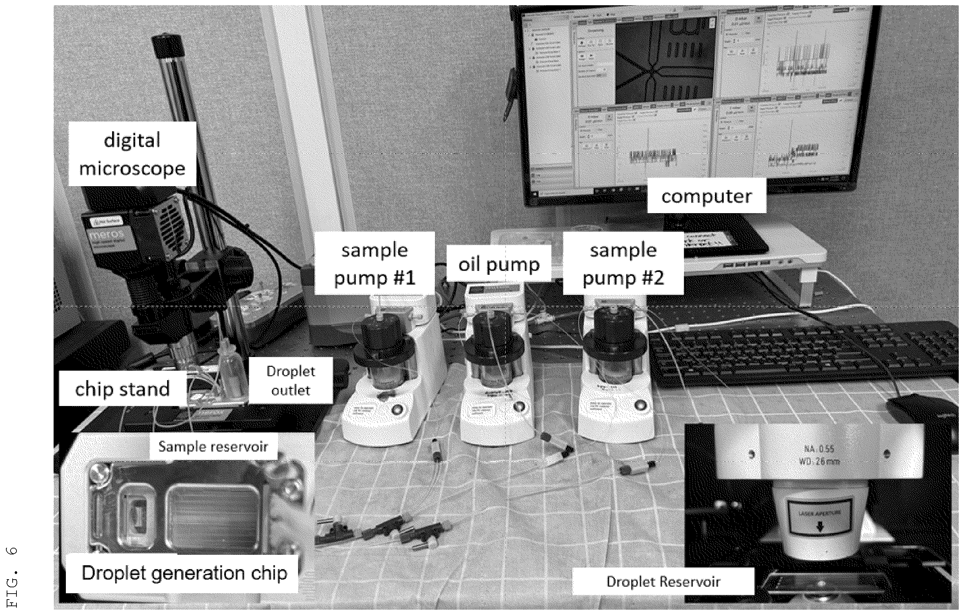

FIG. 6 shows an experimental setup for droplet generation. The setup consists of a digital microscope, a chip stand, an oil pump, two sample pumps, a sample reservoir, a droplet outlet, a droplet chip, a computer, and a droplet reservoir chip. EVs and CLIPs are loaded separately into the two sample pumps, and the FC-40 surfactant is loaded into the oil pump and the sample reservoir. The oil phase pump pushes the FC-40 into the vertical side of the T-junction, and the sample pumps push EVs and CLIPs into the sample channel, and the EVs and CLIPs meet in the T-junction to form water-in-oil droplets, which are then collected at the droplet outlet. Then, the droplets are imaged for analysis by using the droplet reservoir chip under a fluorescence microscope.

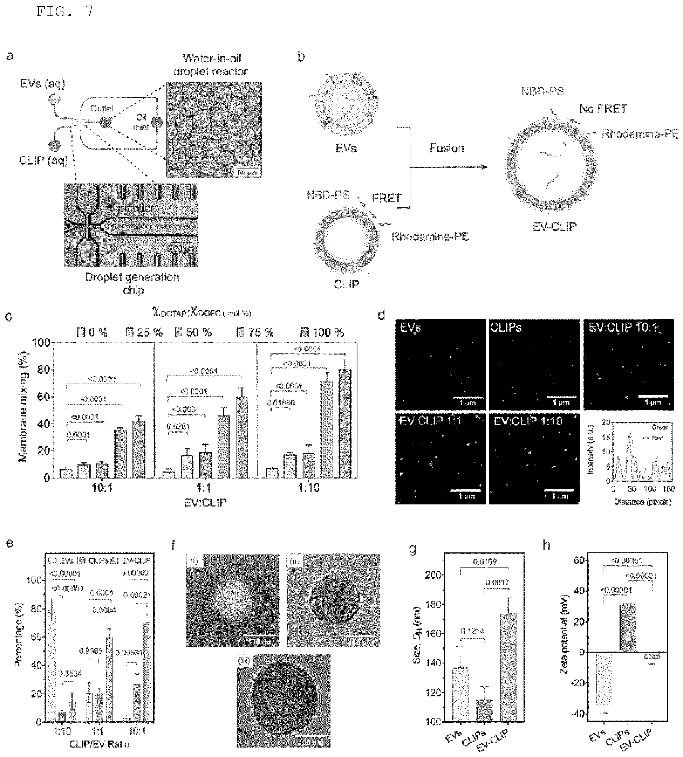

FIG. 7 shows controlled fusion of EVs and CLIPs. (a) Schematic of CLIP and EV fusion using a droplet generation chip. The microfluidic device was designed to generate water-in-oil droplet reactors at the flow-focusing junction, where two aqueous phases (EVs and CLIPs) met and were transferred into an oil stream (surfactant PFPE-PEG in FC-40) to form droplets. (b) Schematic of Forster resonance energy transfer (FRET)-based lipid mixing assay to analyze the fusion reaction of EVs and CLIPs. The average distance between FRET-pair donor (green, nitrobenzoxadiazole) and acceptor (red, rhodamine) lipid probes increases following charge-based fusion of a labeled CLIP membrane and unlabeled EV membrane, resulting in decreased FRET efficiency (PE: phosphoethanolamine; PS: phosphatidylserine). (c) The results of analysis of the extent of lipid mixing in fused vesicles with respect to the fusion ratio of EVs and CLIPs and the percentage of DOTAP in the CLIP composition. All statistical analyses were performed by one-way ANOVA using Dunnett's multiple comparison test. (d) Confocal laser scanning microscope (CLSM) images of EVs, CLIPs, and fused vesicles, indicating colocalization of EVs (green) and CLIPs (red). With high-resolution confocal laser scanning microscope (CLSM) images of EVs, CLIPs, and fused vesicles (EV-CLIP) in three different ratios of EVs to CLIPs (75% DOTAP) (molar ratios 10:1, 1:1, and 1:10). Intensity analysis shows colocalization of EVs (green) and CLIPs (red). (e) A graph showing the ratio of each vesicle in the analysis results of (d). Analysis was conducted from 10 independent images, with vesicle counts ranging from 100 to 160. Statistical analysis was performed by one-way ANOVA. (f) Representative images of a CLIP, an EV, and a fused vesicle taken using the transmission electron microscope (TEM) (diameter of fused vesicle: 174.85±13.83 nm, n=5). scale bar: 100 nm. (g) Size of vesicles before and after controlled fusion (EVs: 137.00±14.30 nm, CLIPs: 115.03±8.86 nm, EV-CLIPs: 174.10±10.28), in which data represent mean±SD, n=3. All statistical analyses were performed by one-way ANOVA using Tukey's multiple comparison test. (h) Zeta potential of vesicles before and after controlled fusion, in which data represent mean±SD, n=3. All statistical analyses were performed by one-way ANOVA using Tukey's multiple comparison test.

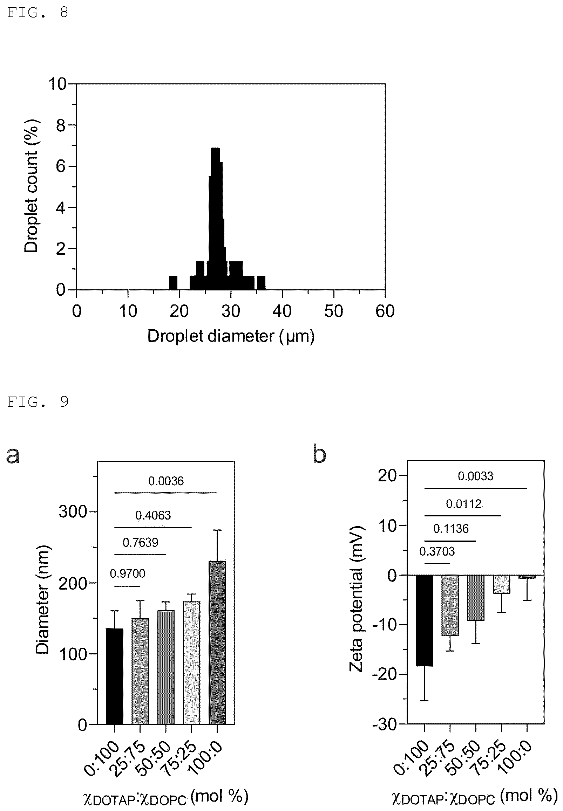

FIG. 8 shows droplets size distribution graph, in which droplets were measured using the Droplet Monitor application. It is confirmed that the average size of droplets was determined to be 27.40±2.06 $\mu$m.

FIG. 9 shows an increase in size and zeta potential of the fused vesicles with respect to the percentage of DOTAP. (a) A graph showing an increase in size of the fused vesicles with respect to the percentage of DOTAP (0% DOTAP: 135.9 ±24.7 nm, 25% DOTAP: 150.5±24.3 nm, 50% DOTAP: 161.6±11.9 nm, 75% DOTAP: 174.1±10.3 nm, 100% DOTAP: 231.3±42.8 nm). Each data point represents mean±SD, n=3. All statistical analyses were performed by one-way ANOVA. (b) A graph showing a change in surface charge of the fused vesicles with respect to the percentage of DOTAP (0% DOTAP: -18.4±6.9, 25% DOTAP: -12.3±3.0, 50% DOTAP: - 9.24±4.55, 75% DOTAP: -3.74±3.80, 100% DOTAP: -0.71±4.39). Each data point represents mean±SD, n=3. All statistical analyses were performed by one-way ANOVA.

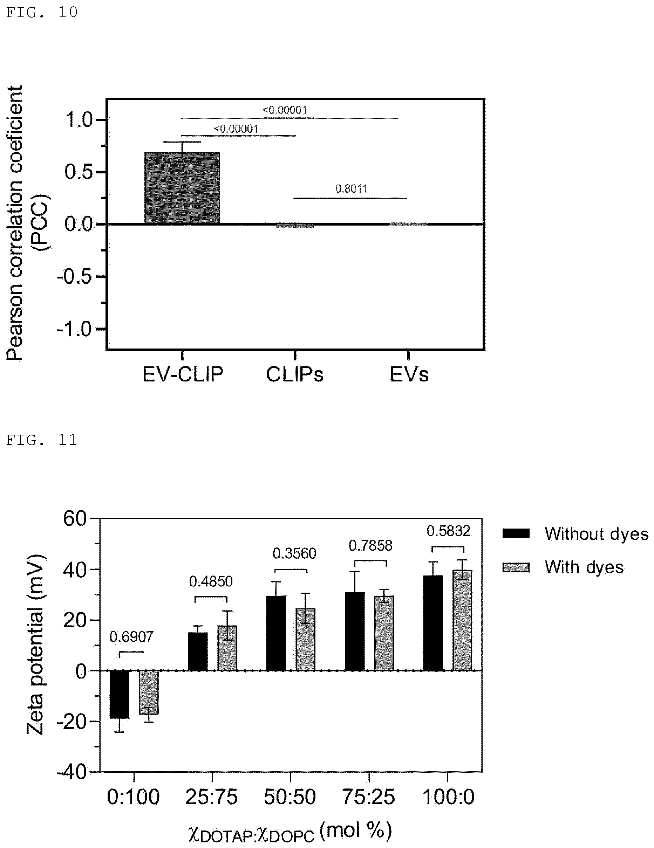

FIG. 10 shows Pearson correlation coefficient (PCC) values of the fused vesicles, liposomes, and EVs. Pearson correlation coefficient (PCC) was measured in 10 different single vesicles and calculated using the JaCoP algorithm in ImageJ. It is confirmed that PCC of the fused vesicles was 0.690±0.096, PCC of liposomes was -0.0139±0.01264, and PCC of EVs was 0.0021±0.0066.

FIG. 11 shows effect of Rho-NBD addition to liposomes in terms of zeta potential. It shows zeta potential changes of liposomes with different ratio of DOTAP (0%, 25%, 75%, and 100% DOTAP). Each data point represents mean±SD, n=3. All statistical analyses were performed using two-tailed unpaired *Student's t*-test.

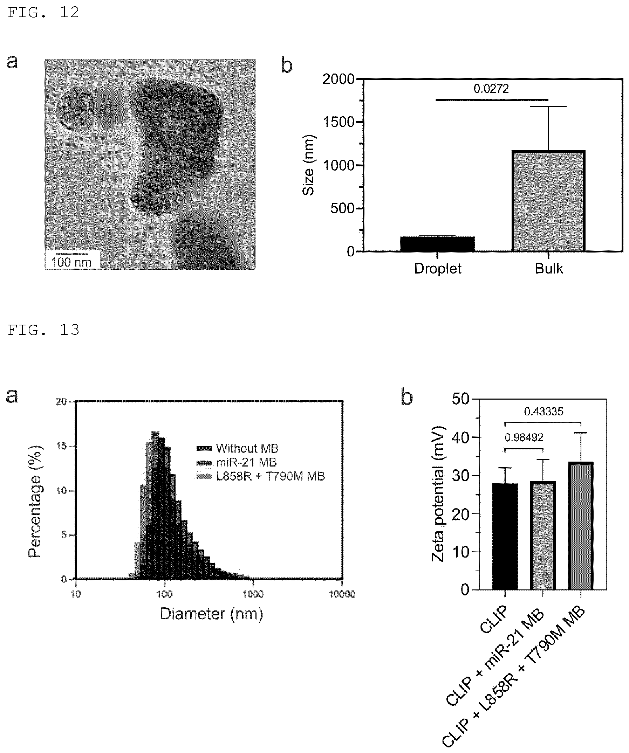

FIG. 12 shows TEM image and size comparison of aggregates formed during EV-liposome fusion in bulk-scale. (a) TEM image showing aggregates formed when EV-liposome fusion occurs in bulk scale. Scale bar: 100 nm. (b) Comparison of the sizes of fused vesicles in droplet and bulk scale. The average size of fused vesicles in droplets is 174.1±10.28 nm, the average size of fused vesicles in bulk scale is 1173.17±508.58 nm. Each data point represents mean±SD, n=3. All statistical analyses were performed using two-tailed unpaired *Student's* t-test.

FIG. 13 shows effect of MB insertion into the liposomes in terms of size and zeta potential. (a) A graph showing size distribution of three different liposome populations (without molecular beacon (MB); with miR-21-detecting MB; and with EGFR mutation-detecting MB). (b) A graph showing zeta potential of three different liposome populations (without molecular beacon (MB); with miR-21 detecting MB; and with EGFR mutation detecting MB). Each data point represents mean±SD, n=3. All statistical analyses were performed by one-way ANOVA.

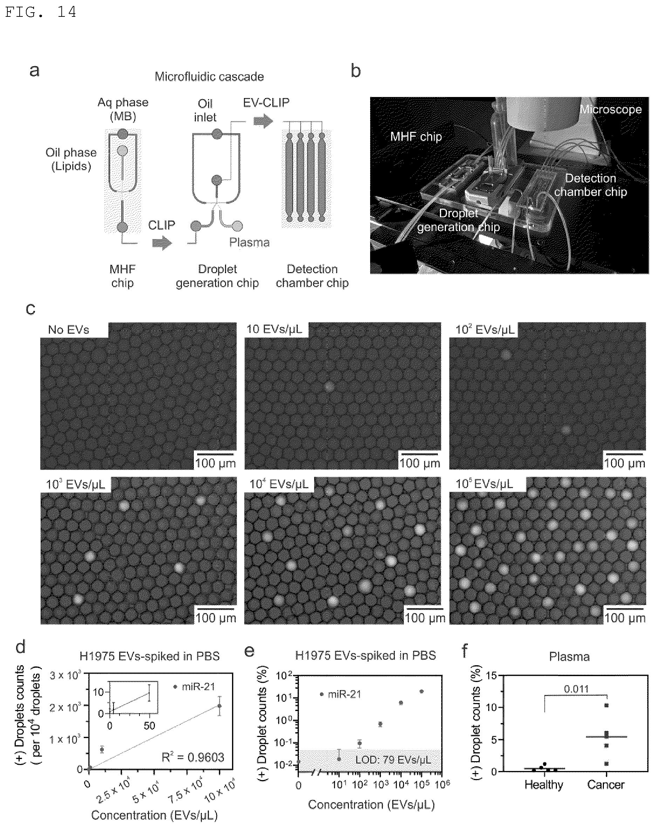

FIG. 14 shows molecular beacon-based tumor EV detection. (a) Schematic of CLIP generation using an MHF chip, fusion reaction with EVs using a droplet reactor, and detection using a detection chamber chip. (b) A photograph showing the three chips. (c) Representative fluorescence images of miR-21 molecular beacon-based detection of various concentrations of H1975 cells-derived EVs in phosphate-buffered saline (PBS). (d) Results of miR-21 detection with respect to the concentration of H1975 cells-derived EVs, data representing mean±SD, n=3, (e) the limit of detection (LOD) was found to be 79 EVs/μl. (f) A graph showing results of miR-21 molecular beacon-based detection in normal persons and cancer patients, confirming differences therebetween.

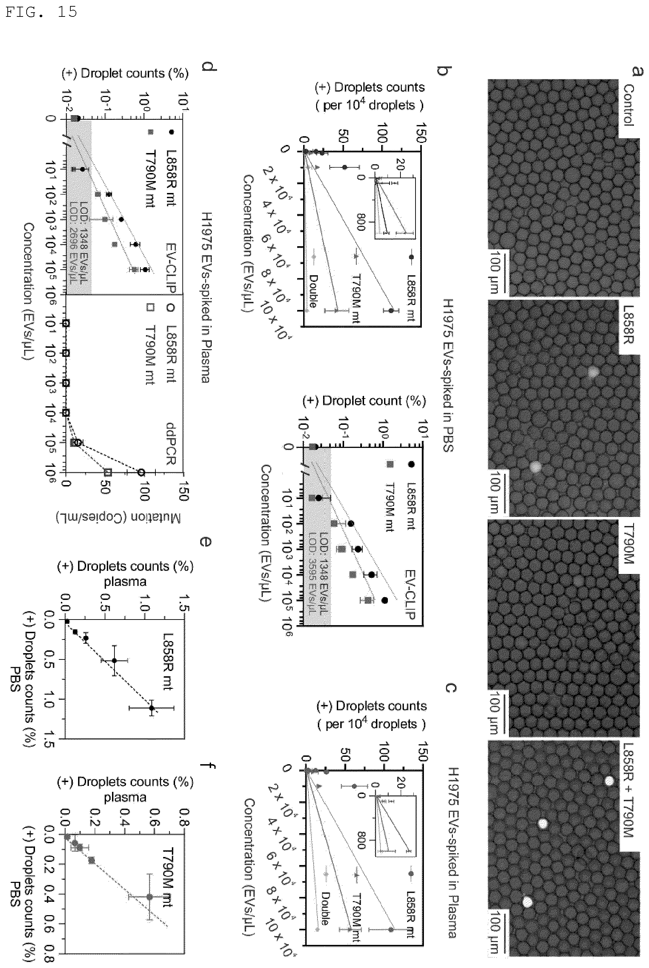

FIG. 15 shows molecular beacon-based tumor EV detection. (a) Representative fluorescence images of L858R, T70M, and L858R+T790M molecular beacon-based detection of H1975 cells-derived EVs in phosphate-buffered saline (PBS). (b) Results of detection of L858R and T790M mutations with respect to the concentration of H1975 cells-derived EVs in phosphate-buffered saline (PBS). Data represent mean±SD, n=3, and limit of detection (LOD) was 1348 EVs/μl and 3595 EVs/μl, respectively. (c) Results of detection of L858R and T790M mutations with respect to the concentration of H1975 cells-derived EVs in human plasma. Data represent mean±SD, n=3, and limit of detection (LOD) was 1348 EVs/μl and 2926 EVs/μl, respectively. (d) Results of Droplet Digital Polymerase Chain Reaction (ddPCR) performed after EV isolation. Each data point represents mean±SD, n=3. (e) and (f) Correlation of the detection readouts of EGFR L858R mutation and T790M mutation in PBS and human plasma, in which data represent mean±SD from independent analysis, n=3.

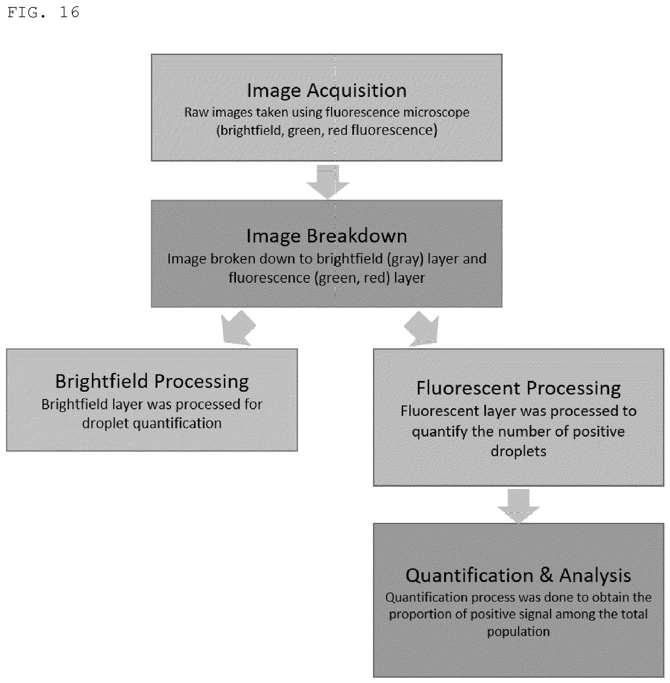

FIG. 16 shows a flowchart of droplet image processing. The flowchart describes the processing steps of droplet image for the analysis, starting from the image acquisition, to processing, quantification and analysis

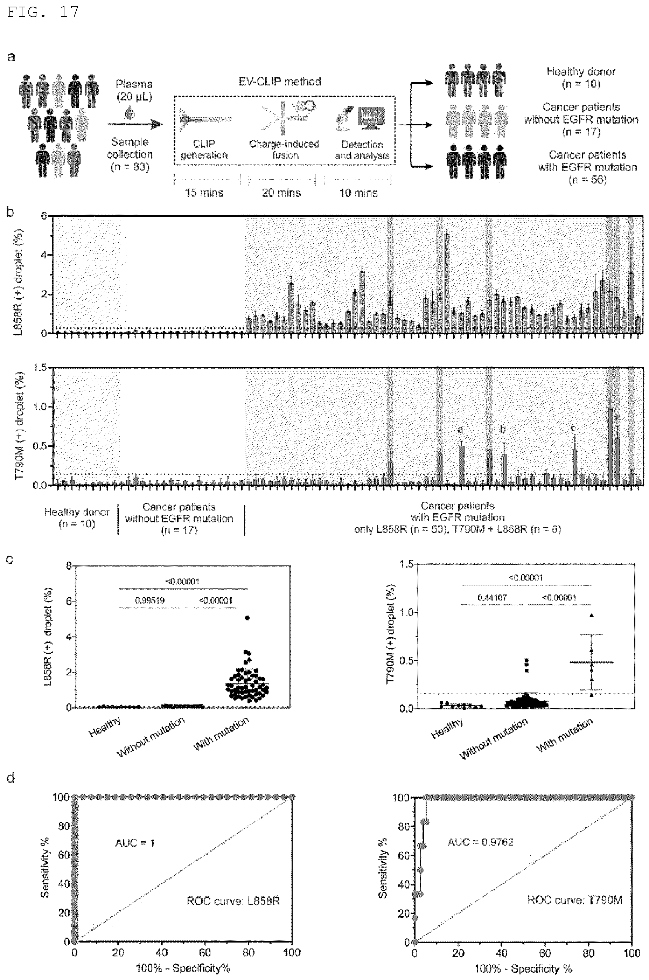

FIG. 17 shows detection of EGFR L858R and T790M mutations from lung cancer patient samples using a fusion system. (a) Schematic of the detection of EGFR L858R and T790M mutations from 10 healthy donors and 73 lung cancer patients using EV-CLIP fusion from plasma samples of normal persons and patients. (b) L858R mutation and T790M mutation droplet detection % from 10 healthy donors, 17 lung cancer patients without EGFR mutation, and 56 lung cancer patients with EGFR mutation. (c) Comparison of detection of L858R and T790M mutations by group. (d) ROC (Receiver Operating Characteristic) curves for detection of L858R and T790M mutations, in which AUC (Area Under the Curve) was shown to be 1.0 for L858R and 0.9762 for T790M. Each data point represents the average of three independent repetitions. All statistical analyses were performed by one-way ANOVA.

## DETAILED DESCRIPTION OF THE INVENTION AND PREFERRED EMBODIMENTS OF THE INVENTION

Unless otherwise defined, all technical and scientific terms used herein have the same meanings as typically understood by those skilled in the art to which the present invention belongs. In general, the nomenclature used herein is well known in the art and is typical.

Investigating protein-free fusion mechanisms in individual extracellular vesicle (EV) and liposome would not only improve understanding of various EV populations, but also allow for the investigation of RNA of single EVs, which may identify distinct subpopulations in disease condition. However, these analytical techniques require EV lysis, which in most cases results in reduced sensitivity, high cost, and labor intensiveness, time-consuming processes. The present inventors developed an approach for detection of individual EV subpopulations based on charge-mediated fusion of EVs and liposomes. The surface charge of liposomes was tuned by changing the ratio of positively and negatively charged lipids, and certain ratios exhibited very efficient and stable fusion to exosomes without compromising membrane properties confirmed by membrane mixing assays. This method leveraging the advantages of high fusion rate, rapid applicability, and broad range of charged-liposome EV detection (EV-CLIP) demonstrates remarkable sensitivity and selectivity for EV-derived RNAs in a lysis-free manner using droplet-microfluidics. In addition, EV-CLIP allows to the digital detection of EGFR L858R and T790M mutations in plasma samples collected from 73 lung cancer patients (17 patients without mutation and 56 patients with mutation) and 10 healthy donors without EV pre-isolation step, further simplifying the detection process and preventing EV loss. Overall, EV-CLIP holds great promise in the clinical setting for accurate quantification of rare EV subpopulations and provides novel opportunities to explore fundamental questions in cancer biology.

Accordingly, an aspect of the present invention relates to a liposome for detecting a cancer cell-derived extracellular vesicle (EV) comprising a cationic lipid and a neutral lipid, in which a cancer cell-specific molecular beacon is encapsulated within the liposome.

As used herein, the term "extracellular vesicle (EV)" refers to a small secretory vesicle (typically about 30-800

EP 4 636 402 A1

nm) that may comprise nucleic acids, proteins, or other biomolecules, and EVs include exosomes and microvesicles. EVs may act as cellular messengers by transporting biomolecules to various locations in living organisms or biological systems.

As used herein, the term "cancer cell-specific molecular beacon" refers to a molecular beacon that specifically binds to a cancer-specific substance that is present in cancer cell-derived extracellular vesicles but not in normal cell-derived extracellular vesicles.

In the present invention, the cancer-specific substance present in cancer cell-derived extracellular vesicles may include microRNA-21 (miR-21), microRNA-25 (miR-25), microRNA-27 (miR-27), microRNA-54 (miR-54), microRNA-155 (miR-155), microRNA-210 (miR-210), microRNA-375 (miR-375), microRNA-451 (miR-451), microRNA-486 (miR-486), microRNA-495 (miR-495), microRNA-574-3p (miR-574-3p), EGFR L858R mutation, EGFR T790M mutation, GPC1, KRAS, AR-V7, survivin, TK-1, c-Myc, GalNAc-T, Cyclin D1, and so on (Wei Pan, et al., Anal. Chem. 2013, 85, 21, 10581-10588; Xiang-Hong Peng, et al., Cancer Res (2005) 65 (5): 1909-1917), but is not limited thereto.

As used herein, the "molecular beacon (MB)" is an oligonucleotide that forms a hairpin-shaped secondary structure with the 3' end tagged with a quencher material, and the molecular beacon probe specifically hybridizes in a region complementary to a template gene during the annealing process, and the distance between the fluorescent material and the quencher material increases, thereby releasing inhibition of luminescence by the quencher material and fluorescing. On the other hand, unhybridized molecular beacons maintain the secondary structure thereof and are therefore inhibited by the quencher and do not fluoresce.

In an embodiment of the present invention, molecular beacons represented by the nucleotide sequences of SEQ ID NO: 1 and SEQ ID NO: 2 were respectively used to detect EGFR L858R and T790M mutations in H1975 lung cancer cell lines, and tumor-derived extracellular vesicles (tEVs) were detected using a molecular beacon represented by the nucleotide sequence of SEQ ID NO: 3, which is designed to target microRNA-21 (or miR-21) known to be upregulated in various tumor types such as breast cancer, colon cancer, lung cancer, pancreatic cancer, prostate cancer, and stomach cancer.

As used herein, the term "lipid" or "lipid analogue" refers to a molecule including at least one hydrophobic moiety or group and optionally also at least one hydrophilic moiety or group. A molecule including hydrophobic and hydrophilic moieties is also often referred to as an amphipathic substance. Lipids are generally not very soluble in water. In an aqueous environment, the amphipathic property allows the molecules to self-assemble into organized structures and different phases. One of these phases, when they exist in vesicles, multilamellar/unilamellar liposomes or membranes in aqueous environments, is composed of a lipid bilayer. Hydrophobic groups include nonpolar groups including, but not limited to, long-chain saturated and unsaturated aliphatic hydrocarbon groups and such groups substituted with at least one aromatic, alicyclic or heterocyclic group(s). Hydrophilic groups may include polar and/or charged groups and may include carbohydrate, phosphate, carboxyl, sulfate, amino, sulfhydryl, nitro, hydroxyl, and other similar groups.

As used herein, the term "amphipathic" molecule refers to a molecule having both polar and nonpolar portions. An amphipathic compound often has a polar head attached to a long hydrophobic tail. In some embodiments, the polar portion is soluble in water, while the nonpolar portion is insoluble in water. Additionally, the polar portion may have a formal positive charge or a formal negative charge. Alternatively, the polar portion may have both formal positive and negative charges and may be a zwitterion or an internal salt. For the purposes of this specification, an amphipathic compound may be one or more natural or non-natural lipids and lipid-like compounds, but not limited thereto.

The term "lipid analogue", "lipid-like compound", or "lipid-like molecule" refers to a substance that is structurally and/or functionally lipid-like but may not be considered a lipid in the strict sense. For example, this term includes compounds capable of forming amphipathic layers when present in vesicles, multilamellar/unilamellar liposomes or membranes in aqueous environments, and includes surfactants or synthetic compounds having both hydrophilic and hydrophobic moieties. Generally, this term means a molecule including hydrophilic and hydrophobic moieties with different structural organizations that may or may not be similar to that of lipids. Herein, the term "lipid" should be construed to encompass both lipids and lipid analogues, unless it is clearly contradictory in context.

As used herein, the term "cationic lipid" or "cationic lipid analogue" refers to a lipid or lipid-like substance having a net positive charge. A cationic lipid or lipid analogue binds to a negatively charged nucleic acid by electrostatic interaction. In general, a cationic lipid has a lipophilic moiety such as a sterol, acyl chain, diacyl or larger acyl chain, and the head group of the lipid is usually positively charged.

In certain embodiments, the cationic lipid or lipid analogue has a net positive charge only at a certain pH, particularly acidic pH, whereas, preferably, at a higher pH, such as physiological pH, it does not have any net positive charge and is uncharged, namely neutral. This ionizable behavior is deemed to enhance efficacy by aiding endosomal escape and reducing toxicity compared to particles that remain cationic at physiological pH.

In the present invention, the cationic lipid is selected from the group consisting of, but is not limited to, 1,2-dioleoyl-3-trimethylammonium propane (DOTAP), 1,2-dioleyloxy-3-dimethylamino-propane (DODMA), 1,2-di-O-octadecenyl-3-trimethylammonium propane (DOTMA), 3β-[N-(N',N'-dimethylaminoethane)carbamoyl]cholesterol (DC-Chol), dimethyldioctadecylammonium bromide (DODAB), 1,2-dioleoyl-3-dimethylammonium propane (DODAP), dioctadecyldimethyl ammonium chloride (DODAC), 1,2-distearyloxy-N,N-dimethyl-3-aminopropane (DSDMA), 1,2-dimyristyloxy-propyl-3-dimethyl-hydroxyethyl ammonium bromide (DMRIE), 1,2-dimyristoyl-sn-glycero-3-ethylphosphocholine (DMEPC), 1,2-dimyristoyl-3-trimethylammonium propane (DMTAP), 1,2-dioleoyl-3-dimethyl-hydroxyethyl ammonium bromide (DORIE), 2,3-dioleyloxy-N-[2-(sperminecarboxamido)ethyl]-N,N-dimethyl-1-propanaminium (DOSPA), 1,2-dilinoleyloxy-N,N-dimethyl-3-aminopropane (DLinDMA), 1,2-dilinolenyloxy-N,N-dimethyl-3-aminopropane (DLenDMA), dioctadecylamidoglycylspermine (DOGS), 3-dimethylamino-2-(cholest-5-en-3-β-oxybutan-4-oxy)-1-(cis,cis-9,12-octadecadienoxy) propane (CLinDMA), 2-[5'-(cholest-5-en-3-β-oxy)-3'-oxapentoxy]-3-dimethyl-1-(cis,cis-9',12'-octadecadienoxy)propane (CpLinDMA), N,N-dimethyl-3,4-dioleyloxybenzylamine (DMOBA), 1,2-N,N'-dioleylcarbamyl-3-dimethylaminopropane (DOcarbDAP), 2,3-dilinoleoyloxy-N,N-dimethylpropylamine (DLinDAP), 1,2-N,N'-dilinoleylcarbamyl-3-dimethylaminopropane (DLincarbDAP), 1,2-dilinoleoylcarbamyl-3-dimethylaminopropane (DLinCDAP), 2,2-dilinoleyl-4-dimethylaminomethyl-[1,3]-dioxolane (DLin-KDMA), 2,2-dilinoleyl-4-dimethylaminoethyl-[1,3]-dioxolane (DLin-K-XTC2-DMA), 2,2-dilinoleyl-4-(2-dimethylaminoethyl)-[1,3]-dioxolane (DLin-KC2-DMA), heptatriaconta-6,9,28,31-tetraen-19-yl-4-(dimethylamino)butanoate (DLin-MC3-DMA), (±)-N-(3-aminopropyl)-N,N-dimethyl-2,3-bis(cis-9-tetradecenyloxy)-1-propanaminium bromide (GAP-DMORIE), (±)-N-(3-aminopropyl)-N,N-dimethyl-2,3-bis(dodecyloxy)-1-propanaminium bromide (GAP-DLRIE), (±)-N-(3-aminopropyl)-N,N-dimethyl-2,3-bis(tetradecyloxy)-1-propanaminium bromide (GAP-DMRIE), (±)-N-(2-aminoethyl)-N,N-dimethyl-2,3-bis(tetradecyloxy)-1-propanaminium bromide, βN-(4-carboxybenzyl)-N,N-dimethyl-2,3-bis(oleyloxy)propan-1-aminium (DOBAQ), 1,2-dimyristoyl-3-dimethylammonium-propane (DMDAP), 1,2-dipalmitoyl-3-dimethylammonium-propane (DPDAP), N1-[2-((1S)-1-[(3-aminopropyl)amino]-4-[di(3-aminopropyl)amino]butylcarboxamido)ethyl]-3,4-di[oleyloxy]-benzamide (MVL5), 1,2-dioleoyl-sn-glycero-3-ethylphosphocholine (DOEPC), 2,3-bis(dodecyloxy)-N-(2-hydroxyethyl)-N,N-dimethylpropan-1-aminium bromide (DLRIE), N-(2-aminoethyl)-N,N-dimethyl-2,3-bis(tetradecyloxy)propan-1-aminium bromide (DMORIE), di((Z)-non-2-en-1-yl) 8,8'-((((2(dimethylamino)ethyl)thio)carbonyl)azanediyl)dioctanoate (ATX), N,N-dimethyl-2,3-bis(dodecyloxy) propan-1-amine (DLDMA), N,N-dimethyl-2,3-bis(tetradecyloxy)propan-1-amine (DMDMA), di((Z)-non-2-en-1-yl)-9-((4-(dimethylaminobutanoyl)oxy)heptadecanedioate (L319), N-dodecyl-3-((2-dodecylcarbamoyl-ethyl)-{2-[(2-dodecylcarbamoyl-ethyl)-2-{(2-dodecylcarbamoyl-ethyl)-[2-(2-dodecylcarbamoyl-ethylamino)-ethyl]-amino}-ethylamino) propionamide (lipidoid 98N12-5), and 1-[2-[bis(2-hydroxydodecyl)amino]ethyl-[2-[4-[2-[bis(2-hydroxydodecyl)amino] ethyl]piperazin-1-yl]ethyl]amino]dodecan-2-ol (lipidoid C12-200).

In the present invention, the liposome may comprise at least one non-cationic lipid such as anionic lipid and/or neutral lipid.

As used herein, the term "anionic lipid" refers to any lipid that is negatively charged at a selected pH. As used herein, the term "neutral lipid" refers to any of a number of lipid species present in either an uncharged or neutral zwitterion form at a selected pH.

In the present invention, the neutral lipid is selected from the group consisting of, but is not limited to, 1,2-dioleoyl-sn-glycero-3-phosphocholine (DOPC), 1,2-dimyristoyl-sn-glycero-3-phosphocholine (DMPC), 1,2-dioleoyl-sn-glycero-3-phosphoethanolamine (DOPE), 1,2-dipalmitoyl-sn-glycero-3-phosphocholine (DPPC), 1,2-distearoyl-sn-glycero-3-phosphocholine (DSPC), 1,2-dilinoleoyl-sn-glycero-3-phosphocholine (DLPC), phosphatidylserine (PS), phosphatidylethanolamine (PE), phosphatidylglycerol (PG), phosphoric acid (PA), and phosphatidylcholine (PC).

Preferably, the cationic lipid is 1,2-dioleoyl-3-trimethylammonium propane (DOTAP), and the neutral lipid is 1,2-dioleoyl-sn-glycero-3-phosphocholine (DOPC), but the present invention is not limited thereto.

In the present invention, the molar ratio (%) of the cationic lipid in the liposome may be 25 to 75%, but is not limited thereto.

In an embodiment of the present invention, when 25% DOTAP was included in the total lipid, the surface charge shifted significantly from negative to positive, and the surface charge was maintained as positive up to 100% (FIG. 1d), and the fusion rate with extracellular vesicles was increased (FIG. 7c). However, the CLIP comprising 100% DOTAP showed instability and formed aggregates over time despite having the highest fusion efficiency (FIGs. 4 and 12). Therefore, the molar ratio (%) of the cationic lipid in the liposome is preferably 75%, and the molar ratio of cationic lipid to neutral lipid is 75:25, but the present invention is not limited thereto.

Another aspect of the present invention relates to a composition for detecting a cancer cell-derived extracellular vesicle comprising the liposome for detecting an extracellular vesicle.

Still another aspect of the present invention relates to a kit for detecting a cancer cell-derived extracellular vesicle comprising the liposome for detecting an extracellular vesicle.

Yet another aspect of the present invention relates to a method of detecting a cancer cell-derived extracellular vesicle comprising fusing the liposome for detecting an extracellular vesicle with an extracellular vesicle derived from a biological sample; and determining that the extracellular vesicle is a cancer cell-derived extracellular vesicle when a fluorescence signal is generated.

In the present invention, the fusion may be performed within a droplet reactor, but the present invention is not limited thereto.

In the present invention, the droplet reactor may comprise two aqueous phase channels, one oil phase channel, one junction, and one outlet channel, and may be characterized in that aqueous droplets are generated at the junction by introducing liposomes and extracellular vesicles derived from a biological sample respectively into the two aqueous phase channels, but the present invention is not limited thereto.

In an embodiment of the present invention, to ensure precise control over stoichiometry of vesicles (EVs and CLIPs) and prevent undesired aggregation, the entire fusion process was performed within the droplet reactor using a $\mu$Encapsulator that allows precise control, minimizing potential aggregation that may occur in bulk-scale reaction (FIG. 6). The $\mu$Encapsulator operates by allowing EVs and CLIPs as separate phases to flow through two aqueous inlets at a flow rate of 1.5 $\mu$l/min, while the continuous oil phase inlet delivers FC-40 as a biocompatible surfactant at a flow rate of 35 $\mu$l/min, enabling generation of aqueous droplets comprising both vesicles when these two vesicles meet at the junction within the oil phase (FIG. 7a).

In the present invention, the molar ratio of liposomes to extracellular vesicles derived from a biological sample may be 1:1 to 10:1, but is not limited to.

In an embodiment of the present invention, when the molar ratio of extracellular vesicles to liposomes was 10:1, the lipid membrane mixing percentage was $35.67\pm1.35\%$, when the molar ratio thereof was 1:1, the percentage was $45.91\pm6.33\%$, and when the molar ratio thereof was 1:10, the percentage was $71.45\pm6.58\%$. Therefore, the molar ratio of extracellular vesicles to liposomes is preferably 1:10, but is not limited thereto.

In an embodiment of the present invention, EGFR L858R and T790M mutations could be digitally detected without EV pre-isolation step via charged-liposome EV detection (EV-CLIP) in plasma samples collected from lung cancer patients and healthy donors, and EV loss was minimized due to simplification of the detection process. By fusing the liposome according to the present invention with the sample-derived EV and determining whether a fluorescence signal is generated, it is possible to easily determine whether the sample is a cancer cell.

As used herein, the term "biological sample" includes a biological fluid sample and a biological tissue sample.

As used herein, the term "biological fluid" refers to any fluid isolated from or derived from organisms, including prokaryotes, eukaryotes, bacteria, fungi, yeast, invertebrates, vertebrates, reptiles, fish, insects, plants, and animals, and may include, but is not limited to, serum, plasma, whole blood, urine, saliva, breast milk, tears, sweat, synovial fluid, cerebrospinal fluid, semen, vaginal fluid, sputum, pleural effusion, lymph, ascites, and amniotic fluid. Bronchial washing fluid and culture fluid obtained from cultured cells (e.g.**,** cell culture supernatant, conditioned culture fluid, cell culture fluid, or cell culture medium) may also be biological fluids.

As used herein, the term "biological tissue" refers to a collection of cells derived from prokaryotes, eukaryotes, bacteria, fungi, yeast, invertebrates, vertebrates, reptiles, fish, insects, plants, or animals. In addition, cultured cells may be biological tissues. Non-limiting examples of the biological tissue sample include surgical samples, biopsy samples, tissues, stool, plant tissue, insect tissue, and cultured cells.

A further aspect of the present invention relates to a composition for diagnosing cancer comprising the liposome for detecting an extracellular vesicle.

Still a further aspect of the present invention relates to a kit for diagnosing cancer comprising the liposome for detecting an extracellular vesicle.

Yet a further aspect of the present invention relates to a method of providing information for cancer diagnosis and a method of diagnosing cancer, comprising fusing the liposome for detecting an extracellular vesicle with an extracellular vesicle derived from a biological sample; and determining that the sample is cancerous when a fluorescence signal is generated.

In the present invention, the fusion may be performed within a droplet reactor comprising two aqueous phase channels, one oil phase channel, one junction, and one outlet channel, but the present invention is not limited thereto.

In an embodiment of the present invention, EGFR L858R and T790M mutations could be digitally detected without EV pre-isolation step via charged-liposome EV detection (EV-CLIP) in plasma samples collected from lung cancer patients and healthy donors. By fusing the liposome according to the present invention with the sample-derived EV and determining whether a fluorescence signal is generated, it is possible to easily determine whether the sample is cancerous. Even in early-stage cancer, mutations may be detected at high levels in a short time without preprocessing, making it easy to distinguish cancer patients from healthy donors, and thus demonstrating the value thereof over conventional methods in converting miRNA from tumor-derived EVs into standard clinical markers for cancer diagnosis.

In the present invention, the cancer may be selected from the group consisting of, but is not limited to, squamous cell carcinoma, small cell lung cancer, non-small cell lung cancer, lung cancer, peritoneal cancer, colon cancer, bile duct tumor, nasopharyngeal cancer, laryngeal cancer, bronchial cancer, thyroid cancer, oral cancer, osteosarcoma, gallbladder cancer, bile duct cancer, kidney cancer, bladder cancer, renal cell cancer, melanoma, brain cancer, glioma, glioblastoma, brain tumor, skin cancer, pancreatic cancer, breast cancer, liver cancer, bone marrow cancer, small intestine cancer, esophageal cancer, large intestine cancer, stomach cancer, eye cancer, urethral cancer, cervical cancer, prostate cancer, ovarian cancer, metastatic cancer, head and neck cancer, rectal cancer, non-Hodgkin's lymphoma, multiple myeloma, acute myelogenous leukemia, lymphoma, acute lymphoblastic leukemia, and chronic myelogenous leukemia.

In the present invention, the diagnostic kit may additionally comprise various components such as one or more types of other component compositions, solutions or devices suitable for the diagnostic method. The diagnostic kit may additionally comprise a compartmented carrier means containing a biological sample, a container containing a reagent, etc. The carrier means is suitable for containing one or more containers, such as bottles or tubes, each container containing independent components used in the method of the present invention. Herein, the required formulation may be easily dispensed into a container by those skilled in the art.

A better understanding of the present invention may be obtained through the following examples. These examples are merely set forth to illustrate the present invention and are not to be construed as limiting the scope of the present invention, as will be apparent to those skilled in the art.

**Example 1: Materials and methods**

Example 1-1: Reagents and equipment

0.2 $\mu$m syringe filter (Minisart® NML Syringe Filters, S6534), 1x phosphate-buffered saline (pH 7.4, Gibco), antibiotic/antimycotic (Gibco, 15240062), anti-CD63 (BD Bioscience, BD556019), anti-CD81 (BD Bioscience, BD555675), anti-CD9 (BD Bioscience, BD555370), anti-EGFR (Abcam, ab192263), EV-depleted FBS (Systems Biosciences Inc), Exodisc™-C (LabSpinner), molecular beacon (Oligo, Macrogen), NBD-PE (N-(7-nitrobenz-2-oxa-1,3-diazol-4-yl)-1,2-dihexadecanoyl-sn-glycero-3-phosphoethanolamine, triethylammonium, salt, ThermoFisher, N360), Rhodamine-DHPE (Lissamine rhodamine B 1,2-dihexadecanoyl-sn-glycero-3-phosphoethanolamine, triethylammonium salt, ThermoFisher, L1392), and TMB solution (Sigma-Aldrich, T0440-100ML).

0.2 $\mu$m syringe filter (Minisart® CA Syringe Filters, 16534K), $\mu$Encapsulator 2 reagent droplet chip (Dolomite Microfluidics 3200529), 100K Amicon centrifugal filter (Merck Millipore, UFC810096), 150 $\mu$m aqueous 5-input chip 3D (Dolomite Microfluidic 3200834), 30 $\mu$m chamber chip (Microfluidic ChipShop, 10001447), 96-well plate (Corning, 3364), confocal laser scanning microscope (Zeiss LSM 780NLO, Zeiss), Malvern Zetasizer (Nano ZS), NanoSight instrument (Nanosight NS500, Malvern Instruments), TECAN M2000 Pro Plate Reader (Tecan), and transmission electron microscope (JEOL, JEM-2100).

Example 1-2: Cell culture

H1975 cells (ATCC) were cultured at 37°C in the presence of 5% $CO_2$ under static conditions in Dulbecco's Modified Eagle Medium (DMEM) (Gibco, 11965092) supplemented with 5% (v/v) FBS (Gibco) and 1% antibiotic/antimycotic (100 U/ml penicillin and 100 mg/ml streptomycin, Gibco, 15240062).

Example 1-3: Isolation of extracellular vesicle from cell culture

Extracellular vesicles were isolated from cell culture medium using a commercialized Exo-Disc platform (Woo, H.K., et al., Acs Nano, 2017. 11(2): p. 1360-1370). 70-80% confluent 10 cm culture dishes of cells were washed twice with 1x phosphate-buffered saline (pH 7.4, Gibco) and then cultured for 48 hours in respective medium supplemented with 5% EV-depleted FBS (Systems Biosciences Inc.) and 1% antibiotic/antimycotic, after which the cell culture supernatant was collected, centrifuged at 300 g for 10 minutes and then at 2,000 g for another 10 minutes to remove cell debris, and passed through a 0.2 $\mu$m syringe filter (Minisart® NML Syringe Filters, S6534). Thereafter, the clarified supernatant was concentrated using an ExoDisc platform, a centrifugal disc equipped with anodized aluminum oxide filters with a pore diameter of 0.02 $\mu$m (Exodisc™-C, LabSpinner). The supernatant was centrifuged at 3,000 rpm (approximately 500 g) and passed through the filter, and 100 $\mu$l of concentrated EVs from the collection chamber was resuspended in 1X PBS with a dilution factor of 2. Thereafter, the isolated EVs were analyzed using a NanoSight instrument and a Malvern Zetasizer to determine the concentration, size, and zeta potential distribution, and the EVs were aliquoted and stored at -80°C until they were used for further experiments.

Example 1-4: Liposome synthesis

Liposomes were synthesized by microfluidic hydrodynamic focusing method using a commercialized platform (Dolomite Microfluidics). Lipid mixtures for liposomes were prepared by dissolving various ratios of 18:1 TAP or DOTAP (1,2-dioleoyl-3-trimethylammonium propane, Avanti 890890P) and 18:1 ($\Delta$9-Cis) PC or DOPC (1,2-dioleoyl-sn-glycero-3-phosphocholine, Avanti 850375P)-0%, 25%, 50%, 75%, and 100% DOTAP-in 1 ml of pure ethanol (Duksan Pure Chemicals, UN1170) filtered using a 0.2 $\mu$m syringe filter (Minisart® CA Syringe Filters, 16534K) to reach a final concentration of 5 mg/ml. CLIP synthesis was performed using a 150 $\mu$m hydrophilic 5-input chip 3D (Dolomite Microfluidic 3200834) by adjusting the flow rate of an aqueous phase (1X phosphate-buffered saline, pH 7.4, Gibco) to be 50 $\mu$l/min and the flow rate of an oil phase (lipid mixture) to be 5 $\mu$l/min. The suspension was collected in a 1.5 ml microcentrifuge tube for 15 minutes, purified using a 100K Amicon centrifugal filter (Merck Millipore, UFC810096) with twice PBS washing, and then analyzed using a NanoSight instrument (Nanosight NS500, Malvern Instruments) and a Malvern Zetasizer (Nano ZS) to determine the concentration, size, and zeta potential distribution. Thereafter, CLIPs were aliquoted and stored at 4°C for further use.

Example 1-5: Microfluidic Aided EV-CLIP fusion

Microfluidic Aided fusion was performed using a commercialized platform (Dolomite Microfluidics). The glass chip (μEncapsulator 2 Reagent Droplet Chip, Dolomite Microfluidics 3200529) consists of four channels (two aqueous phase channels, one oil phase channel, and one outlet channel). Liposomes and extracellular vesicles at the same concentration in PBS were loaded into the aqueous channels of the microfluidic chip, and droplets with a diameter of 30 $\mu$m were generated in the FC-40 (RAN Biosciences, 008-FluoroSurfactant-2wtF-50G) oil phase. The numbers of extracellular vesicles and liposomes can be controlled by varying the initial sample input concentration in the range of 10 to $10^5$ particles/$\mu$l, and the pressure of the pumps were maintained at 2,500 mBar and 1,100 mBar for oil and aqueous phases, respectively. The droplets were collected in microcentrifuge tubes for 20 minutes and stored at 4°C until they were used for further experiments.

Example 1-6: Detection of extracellular vesicle through fusion with MB-CLIP

Detection of extracellular vesicles was performed using the microfluidic aided fusion method described above. Various concentrations of extracellular vesicles (10-$10^5$ particles/$\mu$l) and a fixed concentration of molecular beacon containing CLIPs (miR-21 or EGFR L858R and T790M mutations) both in 1X PBS, were loaded into the aqueous channels of the microfluidic chip, where droplets of 30 $\mu$m in diameter were generated in the FC-40 oil phase. The pressure of the pumps were maintained at 2,500 mBar and 1,100 mBar in oil and aqueous phases, respectively. The droplet solution was collected in 1.5 ml brown microcentrifuge tubes for 20 minutes and stored at 4°C until it is used for further experiments.

Example 1-7: EV-CLIP colocalization experiment

For EV-liposome colocalization studies, EVs and liposomes were labeled with 3,3'-dioctadecyloxacarbocyanine perchlorate (DiO) dye (ThermoFisher Scientific, V22886) and Rhodamine-DHPE (Lissamine rhodamine B 1,2 dihexadecanoyl-sn-glycero-3-phosphoethanolamine, triethylammonium salt) (ThermoFisher, L1392), respectively. For EV labeling, 100 $\mu$l of pre-concentrated EVs from the ExoDisc platform were incubated with 2 $\mu$l of 200 nM DiO dye solution on a shaker at room temperature for 20 minutes. After incubation, EVs were centrifuged at 3,000 rpm and washed twice with 1X PBS. The concentrated EVs from the chamber were resuspended in 1X PBS with a dilution factor of 2. For liposome labeling, 1 mol% Rhodamine-DHPE was incorporated into the lipid mixture. The volume was then adjusted to reach a final lipid concentration of 5 mg/ml. Rho-liposomes were synthesized by the microfluidic hydrodynamic focusing method described above. The synthesized liposomes were purified by washing with PBS using a 100K Amicon centrifugal filter to remove free dyes. The dye-modified liposomes were fused with extracellular vesicles using the microfluidic aided fusion method described above. The fused vesicles were then diluted with 1X PBS and imaged with a confocal laser scanning microscope (Zeiss LSM 780NLO) using a x10 Plan-Apochromat and a 0.45 NA objective lens equipped with an LU-NV laser unit 488 nm (green/Alexa Fluor 488) and 560 nm (red/Cy5). The colocalization analysis was performed using Fiji.

Example 1-8: NBD-PE fusion quantification assay

Fusion quantification was performed using a published method (Murtas, G., Systems and synthetic biology, 2010. 4(2): p. 85-93). 1 mol% of Rhodamine-DHPE (Lissamine rhodamine B 1,2 dihexadecanoyl-sn-glycero-3-phosphoethanolamine, triethylammonium salt) (ThermoFisher, L1392) and NBD-PE (N-(7-nitrobenz-2-oxa-1,3-diazol-4-yl)-1,2-dihexadecanoyl-sn-glycero-3-phosphoethanolamine, triethylammonium salt) (ThermoFisher, N360) were incorporated into the lipid mixture. Thereafter, the volume was adjusted to reach a final lipid concentration of 5 mg/ml. Rho-NBD liposomes were synthesized by the microfluidic hydrodynamic focusing method described above. The synthesized liposomes were purified by washing with PBS using a 100K Amicon centrifugal filter to remove free dyes. The dye-modified liposomes were fused with the extracellular vesicles using the microfluidic aided fusion method described above. The fluorescence signals from the fused vesicles were measured using a spectrophotometer (M2000 Pro, Tecan) with excitation-emission wavelengths of 485/535 nm, respectively. The fusion percentage was calculated using the following formula:

$$Fusion \% = \frac{F - F_{min}}{F_{max} - F_{min}} \times 100\%$$

Example **1-9:** Loading of molecular beacon into the liposomes

The miR-21, EGFR L858R, and EGFR T790M targeting molecular beacons (Oligo, Macrogen) were loaded into the liposomes by incorporating a certain amount of molecular beacons with a total concentration of 1 $\mu$M in 1X PBS. The liposomes were then synthesized using the microfluidic hydrodynamic focusing (MHF) method with a final lipid concentration of 5 mg/ml. The miR-21 targeting beacon was loaded into a batch of liposomes, whereas the EGFR L858R and T790M mutation-specific beacons were simultaneously loaded into a different batch of liposomes. The flow rates of the pumps were maintained at 50 $\mu$l/min and 5 $\mu$l/min for aqueous and oil phases, respectively. Thereafter, the molecular beacon-loaded liposomes were purified by washing twice with PBS using a 100K Amicon centrifugal filter, and then analyzed using a Nanosight instrument and a Malvern Zetasizer to determine the concentration, size, and zeta potential distribution. The liposomes were then stored at 4°C for further use.

Example 1-10: Droplet imaging

For imaging, 6 $\mu$l of the droplet solution was loaded into a 30 $\mu$m chamber chip (Microfluidic ChipShop, 10001447) and the droplets were observed in a confocal laser scanning microscope (Zeiss LSM 780NLO) using a x10 Plan-Apochromat and a 0.45 NA objective lens equipped with an LU-NV laser unit 488 nm (green/Alexa Fluor 488) and 560 nm (red/Cy5).
All images for droplet analysis were processed using Fiji.

Example 1-11: Healthy plasma spiking experiment

Healthy human blood plasma approved by the Institutional Review Board (IRB) was obtained from the Red Cross (UNISTIRB-19-41-C). Human blood plasma was diluted in 1X PBS with a dilution factor of 10 and then spiked with H1975 cell-derived EVs at different concentrations ranging from 10 to $10^5$ particles/$\mu$l. The spiked plasma samples and molecular beacon-loaded CLIPs (miR-21 or EGFR L858R and T790M mutations) were loaded into the droplet chip for microfluidic aided fusion. The pressure of the pumps were maintained at 2,500 mBar and 1,100 mBar for oil and aqueous phases, respectively. The droplets were collected in microcentrifuge tubes for 20 minutes and stored at 4°C until they were used for further analysis.

Example 1-12: EGFR mutation detection in patient sample

A total of 83 samples, including 10 healthy donors, 17 lung cancer samples without mutation (6 stage I, 3 stage II, 3 stage III, and 5 stage IV), and 56 lung cancer samples with EGFR mutation (18 stage I, 6 stage II, 7 stage III, and 25 stage IV), was obtained from Inha University Hospital (2019-11-017), Chonnam National University Hwasun Hospital (CNUHH-2022-021), and Pusan National University Hospital (2106-044-104): all healthy donor plasma samples were provided by Inha University Hospital; one stage II sample and one stage III sample without mutation, and 12 stage I, 5 stage II, 6 stage III, and 16 stage IV samples with EGFR mutation were provided by Chonnam National University Hwasun Hospital; and the remaining samples were provided by Pusan National University Hospital. 20 $\mu$l of the patient samples and the CLIPs containing molecular beacons detecting EGFR L858R and T790M were loaded into the droplet chip for microfluidic aided fusion. The pressure of the pumps were maintained at 2,500 mBar and 1,100 mBar for oil and aqueous phases, respectively. The droplets were collected in microcentrifuge tubes for 20 minutes and stored at 4°C until they were used for further analysis.

Example 1-13: Statistical analysis

The experiments were performed independently at least three times, and the number of independent replicates used for each experiment is indicated in each figure legend. Comparisons between two groups were performed by a two-tailed unpaired *Student's* t-test, and comparisons among three or more groups were performed by one-way ANOVA. All statistical analyses were performed in Graphpad Prism 9.3.1.

**Example 2: High-throughput charge-tuning of liposomes**

In the present invention, two types of lipids, cationic 1,2-dioleoyl-3-trimethylammonium propane (DOTAP) and zwitterionic 1,2-dioleoyl-sn-glycero-3-phosphocholine (DOPC), were utilized as building blocks for the preparation of charged liposomes (CLIPs) to achieve high-throughput charge-tuning of liposomes. By utilizing microfluidic hydrodynamic focusing (MHF) for liposome formulation (Jahn, A., et al., J Am Chem Soc 2004, 126 (9), 2674-5), variation in lipid composition were successfully achieved, thus efficiently and rapidly tuning the surface charge of CLIPs (FIGs. 1a, 1b, and 2). The microfluidic device facilitated the CLIP formation by allowing a lipid solution (composed of DOTAP and DOPC) dissolved at a concentration of 5 mg/ml in ethyl alcohol to flow through the central inlet channel at a rate of 5 $\mu$l/min, and an aqueous solution to flow at a rate of 50 $\mu$l/min through two lateral inlet channels, resulting in successful formation of CLIPs. FIG. 1c shows that the round morphology of the liposomes confirmed by a transmission electron microscope (TEM) (diameter: 92.5$\pm$5.65 nm) is consistent with the size measured via dynamic light scattering (DLS) and nanoparticle tracking analysis (NTA) (FIG. 3). As shown in FIG. 1d, the surface charge of CLIPs was tuned from -18.63$\pm$0.76 mV to +38.67$\pm$2.51 mV by changing the composition of the building block lipids, DOTAP and DOPC. Ideally, the higher the ratio of DOTAP, the more positive the surface charge of CLIPs, and vice versa. By adjusting the ratio of DOTAP in the lipids from 0% (pure DOPC) to 100% (pure DOTAP), a noticeable shift in the surface charge from negative to positive was observed when 25% DOTAP was incorporated into the total lipid amount. This change was consistent with the predicted results, and the surface charge was found to increase proportionally with an increase in the ratio of DOTAP (FIG. 1d). In particular, after storing the liposomes for 48 hours, the size of the liposomes with 0%, 25%, 50%, and 75% DOTAP remained stable over time. However, the liposomes with 100% DOTAP continuously increased in size up to 300 nm, and specifically, showed about three times increase in size to 311.50$\pm$8.51 nm (FIGs. 1e and 4).

**Example 3: Charge-induced fusion of liposomes**

After fine-tuning the surface charge of liposomes, the effect on fusion of extracellular vesicles and CLIPs was determined. First, a rapid, label-free, and high-yield method was performed to isolate EVs from H1975 cells using a lab-on-a-disc system integrated with nanofilters (ExoDisc) (FIG. 5a), and EVs with a size of 20-200 nm could be concentrated within 30 minutes using cell culture supernatant (CCS) as a starting material in a tabletop-sized centrifugal microfluidic system (FIG. 5b). The homogeneous size distribution of EVs was confirmed by utilizing nanoparticle tracking analysis (NTA) and dynamic light scattering (DLS) (FIGs. 5c and 5d).

Next, to ensure precise control over stoichiometry of vesicles (EVs and CLIPs) and prevent undesired aggregation, the entire fusion process was conducted within a droplet reactor using a $\mu$Encapsulator that allows precise control, minimizing potential aggregation that could occur in bulk-scale reactions (FIG. 6). The $\mu$Encapsulator operates by allowing EVs and CLIPs as separate phases to flow through two aqueous inlets at a flow rate of 1.5 $\mu$l/min, while the continuous oil phase inlet delivers the biocompatible surfactant FC-40 at a flow rate of 35 $\mu$l/min, enabling the generation of aqueous droplets containing both vesicles when these two vesicles meet at the junction within the oil phase (FIG. 7a). The amounts of EVs and CLIPs contained in one droplet can be controlled by adjusting the initial input concentration of the vesicles according to the Poisson distribution calculation (Table 1).

[Table 1]

| Poisson distribution calculation for EV distribution in individual droplets | | | | | | |
|---|---|---|---|---|---|---|
| Concentration (particles/ml) | Average # of EV / droplet | Probability of a droplet containing (%) | | | | |
| | | 0 EV | 1 EV | 2 EVs | 3 EVs | 4 EVs |
| $10^4$ | 0.00007 | 99.993 | 0.007 | 0 | 0 | 0 |
| $10^5$ | 0.0007 | 99.93 | 0.07 | 0 | 0 | 0 |
| $10^6$ | 0.007 | 99 | 1 | 0 | 0 | 0 |
| $10^7$ | 0.07 | 93 | 7 | 0 | 0 | 0 |
| $10^8$ | 0.7 | 49 | 35 | 12 | 3 | 1 |

Table 1 describes the probability (%) of a droplet containing a certain number of EVs in the concentration range of $10^4$ to $10^8$ particles/ml.

In this microfluidic setup, the initial input concentration of EVs (ranging from 10 to $10^5$ EVs/$\mu$l) and the ratio thereof with CLIPs were controlled within a single droplet having a size of 27.40$\pm$2.06 $\mu$m (FIG. 8). As shown in FIG. 7f, the regular morphology of individuals, semi-fused vesicles and fused vesicles was confirmed through TEM analysis. Additionally, DLS analysis showed that the size of the vesicle population increased after fusion, which is consistent with the observed results (FIG. 7g). However, when the ratio of DOTAP in CLIPs for EV fusion was gradually increased, the size of the fused vesicles remained unchanged until reaching 75% DOTAP, beyond which a significant increase in the vesicle size was observed (FIG. 9a). The confirmation of vesicle fusion was further supported by observing the changes in the surface charge of the fused vesicles using a Zeta sizer, and increased zeta potential correlated with a higher DOTAP ratio, validating the occurrence of fusion (FIG. 9b) .

In order to visualize EV-CLIP fusion events, EVs were labeled with green fluorescent dye (3,3'-dioctadecyloxacarbocyanine perchlorate (DiO) dye (ThermoFisher Scientific, V22886)) and CLIPs were labeled with red fluorescent dye (Rhodamine-DHPE (Lissamine rhodamine B 1,2 dihexadecanoyl-sn-glycero-3-phosphoethanolamine, triethylammonium salt)), and confocal laser scanning microscope (CLSM) imaging was performed. CLSM imaging showed the colocalization of dyes within the fused vesicles, consistent with the TEM images and DLS experiments (FIGs. 7d and 7e). In addition, a Forster resonance energy transfer (FRET)-based lipid mixing assay (Stryer, L.; Haugland, R. P., Proc Natl Acad Sci U S A 1967, 58(2), 719-26) was utilized to evaluate the fusion efficiency, CLIPs were dual-labeled with nitro-2,1,3-benzoxadiazole-4-yl (NBD) as a donor and Lissamine rhodamine B (Rho) as an acceptor, and energy transfer occurred from the excited NBD donor to the Rho acceptor when they were in proximity (Francois-Martin, C.; Pincet, F., Scientific Reports 2017, 7(1), 43860) (FIGs. 7b and 7c). Initially, the effect of dual-labeled lipids on the zeta potential of CLIPs was examined, indicating no significant effect on the surface charge (FIG. 11).

Using the FRET-based assay, an increase in the fusion percentage corresponding to the percentage of DOTAP was observed (FIG. 7c). However, CLIPs containing 100% DOTAP exhibited instability and formed aggregates over time despite having the highest fusion efficiency (FIG. 4). When the fusion method in the droplet reactor was compared with the bulk scale, potential aggregates were revealed (FIG. 12). Consequently, 75% DOTAP was selected as the formulation for subsequent experiments. Also, the ratio of CLIPs to EVs, capable of confirming a maximum fusion percentage of 10:1 (74.96±2.63%), which was further confirmed by CLSM imaging, was adjusted (FIG. 7c).

**Example 4: Digital detection of tumor-derived EVs miRNAs**

After successfully demonstrating efficient fusion between CLIPs and EVs, fluorescence response was directly investigated during the fusion process between molecular beacon (MB)-encapsulated CLIP and tumor-derived EV (tEV) miR-21 using a confocal laser scanning microscope (CLSM) to evaluate the effect of the charged-fusion strategy for detecting EV miRNA in natural environments. In this example, the MB utilized a hairpin structure comprising single-stranded oligonucleotide probes inducing fluorescence quenching characterized by a cyanine 3 fluorophore at the 5' end, a tetrachlorofluorescein quencher at the 3' end, and complementary bases at both ends that bring the fluorophore and the quencher into close proximity (Zhang, P., et al., Angew Chem Int Ed Engl 2001, 40 (2), 402-405) (Table 2).

[Table 2]

| Molecular beacon sequences used for miR-21, EGFR L858R, and T790M mutations | | |
|---|---|---|
| Molecular beacon (MB) | Sequence | SEQ ID NO: |
| EFGR (p.T790M mutation) | AGCTGC/iCy5/ATGATGAGCTGCACGGTGGCAGCTCATCAT/BHQ2 | 1 |
| EFGR (p.L858R mutation) | TTGGCC/iCy3/CGCCCAAAATCTGTGATTAGATTTGGGCG/BHQ2 | 2 |
| miR-21 | TCAACA/iCy3/TCAGTCTGATAAGCTAGTATTATCAGACTGA/BHQ2 | 3 |

Respective underlined parts in Table 2 are the fluorophore and the quencher. The sequence can be referred to Hu, J., et al., Biomaterials, 2018. 183: p. 20-29.

Initially, tumor-derived extracellular vesicles (tEVs) were detected using a molecular probe (MB) specifically designed to target microRNA-21 (or miR-21), which is known to be upregulated in various tumor types, such as breast cancer, colon cancer, lung cancer, pancreatic cancer, prostate cancer, and stomach cancer (Krichevsky, A. M.; Gabriely, G., J Cell Mol Med 2009, 13(1), 39-53). During the generation of CLIPs using the microfluidic hydrodynamic focusing method mentioned above, the miR-21-specific MB (miR-21 MB) was loaded by incorporating 1 $\mu$M of miR-21 MB into the aqueous phase, and this insertion did not significantly affect the size or zeta potential of the liposomes (FIG. 13b). As a proof of concept, EVs were fused with CLIPs containing miR-21 MB in a droplet reactor and detection of H1975 lung cancer cell line-derived EVs was initiated in phosphate-buffered saline (PBS). The concentration of CLIPs was kept constant throughout the experiment, and varying the concentration of H1975 EVs from 10 to $10^5$ EVs/$\mu$l proportionally increased the number of droplets from which fluorescence signals were detected as analyzed through a series of image processing steps (FIGs. 14a-14e).

In order to evaluate system performance, the ability to detect EGFR L858R and T790M mutations (Zhao, B. X., et al., Mol Med Rep 2015, 11(4), 2767-74) in the H1975 lung cancer cell line was determined. To achieve this, EGFR L858R and T790M mutation-specific MB sequences (Hu, J., et al., Biomaterials 2018, 183, 20-29) were introduced by incorporating 1 $\mu$M of each beacon into the aqueous phase using microfluidic hydrodynamic focusing through the same method used to load miR-21 MB. In particular, incorporation of the beacon did not significantly affect the size or zeta potential of CLIPs (FIG. 13).

Next, under the same experimental conditions as the previous setup for the droplet reactor generated by the μEncapsulator, with the only difference being the inclusion of specific beacons targeting EGFR L858R and T790M mutations in CLIPs, these mutations were successfully detected. Fluorescence signals were observed only in the presence of target EVs (FIG. 15). Prior to detecting EGFR mutation in blood plasma samples from cancer patients, detection of EGFR L858R and T790M mutations was demonstrated by introducing various concentrations of H1975-derived EVs into healthy human blood plasma (FIGs. 15b-15d). The concentration range was 10 to $10^5$ EVs/μl, and the detection results for both mutations were consistent with those obtained in PBS (FIGs. 15e and 15f), confirming the robustness of the CLIP and EV fusion system. Remarkably, this system is capable of detecting mutation at a level as low as a single EV per 100 nanoliters, which represents at least about 100 times greater sensitivity than conventional methods (FIG. 15d).

**Example 5**: **Clinical applicability of EV-CLIP (charged-liposome EV detection) for patient sample analysis**

Ultimately, the inventors aimed to put the EV-CLIP system to practical use by testing for EGFR L858R and T790M mutations in blood plasma clinical samples collected from lung cancer patients (FIG. 17a). Plasma samples from 10 healthy donors and 73 lung cancer patients were included (Table 3).

[Table 3]

| Patient Information | | Normal persons (n=10) | Patients without EGFR mutation (n=17) | Patients with EGFR mutation (n=56) |
|---|---|---|---|---|
| Feature | | | | |
| Gender | Female | 5 | 9 | 32 |
| | Male | 5 | 8 | 24 |
| Age | >60 | 4 | 16 | 8 |
| | ≤60 | 6 | 1 | 48 |
| Histological type | Adenocarcinoma | 0 | 17 | 53 |
| | Non-adenocarcinoma | 0 | 0 | 3 |
| Smoking status | Non-smoker | 7 | 0 | 18 |
| | Smoker | 3 | 0 | 11 |
| | Unknown | 0 | 17 | 27 |
| Mutation | L858R | | 0 | 50 |
| | L858R+T790M | | 0 | 6 |
| Disease stage | I | 0 | 6 | 18 |
| | II | 0 | 3 | 6 |
| | III | 0 | 3 | 7 |
| | IV | 0 | 5 | 25 |

Table 3 shows the basic information of 10 healthy donors and 73 patients used in Examples, including gender, age, histological type, smoking status, mutation, and disease stage: 10 healthy donors, 17 cancer patients without mutation (6 stage I, 3 stage II, 3 stage III, and 5 stage IV), and 56 cancer patients with mutation (18 stage I, 6 stage II, 7 stage III, and 25 stage IV). The samples were provided by Inha University Hospital, Chonnam National University Hwasun Hospital, and Pusan National University Hospital after receiving approval from the Institutional Review Board.

Among lung cancer patients, 17 patients had no mutation and 56 patients had mutations. The detection process was performed without any sample preprocessing. Interestingly, the signal obtained from blood plasma of cancer patients had both L858R and T790M mutations was much higher than the threshold obtained from the ROI curve (FIG. 17d). These results suggest a strong correlation between the presence of these mutations and elevated signal levels in the EV-CLIP system. Regardless of cancer stage, it was observed that only cancer patient samples with positive mutation showed significantly elevated signals. This EV-CLIP method is capable of detecting mutation at a high level in a short time without preprocessing even in early-stage cancer, making it easy to distinguish between cancer patients and healthy donors, as well as to diagnose drug resistance in cancer patients at an early stage.

## INDUSTRIAL APPLICABILITY

According to the present invention, charged-liposome EV detection (EV-CLIP) can be very usefully applied in clinical settings by providing accurate quantification of rare EV subpopulations and opening new avenues for exploring fundamental questions in cancer biology. In addition to direct applications in diagnostics, such a fusion system has tremendous potential for a variety of implementations, including drug delivery carrier development, EV marker profiling, and many other possibilities.

Having described certain parts of the present invention in detail above, it will be obvious to those skilled in the art that these specific descriptions are only preferred embodiments, and the scope of the present invention is not limited thereby. Accordingly, the substantial scope of the present invention will be defined by the appended claims and equivalents thereto.

## SEQUENCE LISTING FREE TEXT

An electronic file is attached.

## Claims

1. A liposome for detecting a cancer cell-derived extracellular vesicle (EV) comprising a cationic lipid and a neutral lipid, in which a cancer cell-specific molecular beacon is encapsulated within the liposome.

2. The liposome according to claim 1, wherein the cationic lipid is selected from the group consisting of 1,2-dioleoyl-3-trimethylammonium propane (DOTAP), 1,2-dioleyloxy-3-dimethylamino-propane (DODMA), 1,2-di-O-octadecenyl-3-trimethylammonium propane (DOTMA), 3β-[N-(N',N'-dimethylaminoethane)-carbamoyl]cholesterol (DC-Chol), dimethyldioctadecylammonium bromide (DODAB), 1,2-dioleoyl-3-dimethylammonium-propane (DODAP), dioctadecyldimethyl ammonium chloride (DODAC), 1,2-distearyloxy-N,N-dimethyl-3-aminopropane (DSDMA), 1,2-dimyristyloxy-propyl-3-dimethyl-hydroxyethyl ammonium bromide (DMRIE), 1,2-dimyristoyl-sn-glycero-3-ethylphosphocholine (DMEPC), 1,2-dimyristoyl-3-trimethylammonium propane (DMTAP), 1,2-dioleoyl-3-dimethyl-hydroxyethyl ammonium bromide (DORIE), 2,3-dioleyloxy-N-[2-(sperminecarboxamido)ethyl]-N,N-dimethyl-1-propanaminium (DOSPA), 1,2-dilinoleyloxy-N,N-dimethyl-3-aminopropane (DLinDMA), 1,2-dilinolenyloxy-N,N-dimethyl-3-aminopropane (DLenDMA), dioctadecylamidoglycylspermine (DOGS), 3-dimethylamino-2-(cholest-5-en-3-β-oxybutan-4-oxy)-1-(cis,cis-9,12-octadecadienoxy)propane (CLinDMA), 2-[5'-(cholest-5-en-3-β-oxy)-3'-oxapentoxy]-3-dimethyl-1-(cis,cis-9',12'-octadecadienoxy)propane (CpLinDMA), N,N-dimethyl-3,4-dioleyloxybenzylamine (DMOBA), 1,2-N,N'-dioleylcarbamyl-3-dimethylaminopropane (DOcarbDAP), 2,3-dilinoleoyloxy-N,N-dimethylpropylamine (DLinDAP), 1,2-N,N'-dilinoleylcarbamyl-3-dimethylaminopropane (DLincarbDAP), 1,2-dilinoleoylcarbamyl-3-dimethylaminopropane (DLinCDAP), 2,2-dilinoleyl-4-dimethylaminomethyl-[1,3]-dioxolane (DLin-KDMA), 2,2-dilinoleyl-4-dimethylaminoethyl-[1,3]-dioxolane (DLin-K-XTC2-DMA), 2,2-dilinoleyl-4-(2-dimethylaminoethyl)-[1,3]-dioxolane (DLin-KC2-DMA), heptatriaconta-6,9,28,31-tetraen-19-yl-4-(dimethylamino)butanoate (DLin-MC3-DMA), (±)-N-(3-aminopropyl)-N,N-dimethyl-2,3-bis(cis-9-tetradecenyloxy)-1-propanaminium bromide (GAP-DMORIE), (±)-N-(3-aminopropyl)-N,N-dimethyl-2,3-bis(dodecyloxy)-1-propanaminium bromide (GAP-DLRIE), (±)-N-(3-aminopropyl)-N,N-dimethyl-2,3-bis(tetradecyloxy)-1-propanaminium bromide (GAP-DMRIE), (±)-N-(2-aminoethyl)-N,N-dimethyl-2,3-bis(tetradecyloxy)-1-propanaminium bromide (βAE-DMRIE), N-(4-carboxybenzyl)-N,N-dimethyl-2,3-bis(oleoyloxy)propan-1-aminium (DOBAQ), 1,2-dimyristoyl-3-dimethylammonium-propane (DMDAP), 1,2-dipalmitoyl-3-dimethylammonium-propane (DPDAP), N1-[2-((1S)-1-[(3-aminopropyl)amino]-4-[di(3-aminopropyl)amino]butylcarboxamido)ethyl]-3,4-di[oleyloxy]-benzamide (MVL5), 1,2-dioleoyl-sn-glycero-3-ethylphosphocholine (DOEPC), 2,3-bis(dodecyloxy)-N-(2-hydroxyethyl)-N,N-dimethylpropan-1-aminium bromide (DLRIE), N-(2-aminoethyl)-N,N-dimethyl-2,3-bis(tetradecyloxy)propan-1-aminium bromide (DMORIE), di((Z)-non-2-en-1-yl) 8,8'-((((2(dimethylamino)ethyl)thio)carbonyl)azanediyl)dioctanoate (ATX), N,N-dimethyl-2,3-bis(dodecyloxy)propan-1-amine (DLDMA), N,N-dimethyl-2,3-bis(tetradecyloxy)propan-1-amine (DMDMA), di((Z)-non-2-en-1-yl)-9-((4-(dimethylaminobutanoyl)oxy)heptadecanedioate (L319), N-dodecyl-3-((2-dodecylcarbamoyl-ethyl)-{2-[(2-dodecylcarbamoyl-ethyl)-2-{(2-dodecylcarbamoyl-ethyl)-[2-(2-dodecylcarbamoyl-ethylamino)-ethyl]-amino}-ethylamino)propionamide (lipidoid 98N12-5), and 1-[2-[bis(2-hydroxydodecyl)amino] ethyl-[2-[4-[2-[bis(2-hydroxydodecyl)amino]ethyl]piperazin-1-yl]ethyl]amino]dodecan-2-ol (lipidoid C12-200).

3. The liposome according to claim 1, wherein the neutral lipid is selected from the group consisting of 1,2-dioleoyl-sn-glycero-3-phosphocholine (DOPC), 1,2-dimyristoyl-sn-glycero-3-phosphocholine (DMPC), 1,2-dioleoyl-sn-glycero-3-phosphoethanolamine (DOPE), 1,2-dipalmitoyl-sn-glycero-3-phosphocholine (DPPC), 1,2-distearoyl-sn-glycero-3-phosphocholine (DSPC), 1,2-dilinoleoyl-sn-glycero-3-phosphocholine (DLPC), phosphatidylserine (PS), phosphatidylethanolamine (PE), phosphatidylglycerol (PG), phosphoric acid (PA), and phosphatidylcholine (PC).

4. The liposome according to claim 1, wherein the cationic lipid is 1,2-dioleoyl-3-trimethylammonium propane (DOTAP) and the neutral lipid is 1,2-dioleoyl-sn-glycero-3-phosphocholine (DOPC).

5. The liposome according to claim 1, wherein a molar ratio (%) of the cationic lipid in the liposome is 25 to 75%.

6. A composition for detecting a cancer cell-derived extracellular vesicle comprising the liposome according to claim 1.

7. A kit for detecting a cancer cell-derived extracellular vesicle comprising the liposome according to claim 1.

8. A method of detecting a cancer cell-derived extracellular vesicle, comprising:

fusing the liposome according to claim 1 with an extracellular vesicle derived from a biological sample; and determining that the extracellular vesicle is a cancer cell-derived extracellular vesicle when a fluorescence signal is generated.

9. The method according to claim 8, wherein the fusing is performed within a droplet reactor comprising two aqueous phase channels, one oil phase channel, one junction, and one outlet channel.

10. The method according to claim 9, wherein aqueous droplets are generated at the junction by introducing the liposome and the extracellular vesicle derived from the biological sample respectively into the two aqueous phase channels.

11. A composition for diagnosing cancer comprising the liposome according to claim 1.

12. The composition according to claim 11, wherein the cancer is selected from the group consisting of squamous cell carcinoma, small cell lung cancer, non-small cell lung cancer, lung cancer, peritoneal cancer, colon cancer, bile duct tumor, nasopharyngeal cancer, laryngeal cancer, bronchial cancer, thyroid cancer, oral cancer, osteosarcoma, gallbladder cancer, bile duct cancer, kidney cancer, bladder cancer, renal cell cancer, melanoma, brain cancer, glioma, glioblastoma, brain tumor, skin cancer, pancreatic cancer, breast cancer, liver cancer, bone marrow cancer, small intestine cancer, esophageal cancer, large intestine cancer, stomach cancer, eye cancer, urethral cancer, cervical cancer, prostate cancer, ovarian cancer, metastatic cancer, head and neck cancer, rectal cancer, non-Hodgkin's lymphoma, multiple myeloma, acute myelogenous leukemia, lymphoma, acute lymphoblastic leukemia, and chronic myelogenous leukemia.

13. A kit for diagnosing cancer comprising the liposome according to claim 1.

14. The kit according to claim 13, wherein the cancer is selected from the group consisting of squamous cell carcinoma, small cell lung cancer, non-small cell lung cancer, lung cancer, peritoneal cancer, colon cancer, bile duct tumor, nasopharyngeal cancer, laryngeal cancer, bronchial cancer, thyroid cancer, oral cancer, osteosarcoma, gallbladder cancer, bile duct cancer, kidney cancer, bladder cancer, renal cell cancer, melanoma, brain cancer, glioma, glioblastoma, brain tumor, skin cancer, pancreatic cancer, breast cancer, liver cancer, bone marrow cancer, small intestine cancer, esophageal cancer, large intestine cancer, stomach cancer, eye cancer, urethral cancer, cervical cancer, prostate cancer, ovarian cancer, metastatic cancer, head and neck cancer, rectal cancer, non-Hodgkin's lymphoma, multiple myeloma, acute myelogenous leukemia, lymphoma, acute lymphoblastic leukemia, and chronic myelogenous leukemia.

15. A method of providing information for diagnosing cancer, comprising:

fusing the liposome according to claim 1 with an extracellular vesicle derived from a biological sample; and determining that the sample is cancerous when a fluorescence signal is generated.

16. The method according to claim 15, wherein the fusing is performed within a droplet reactor comprising two aqueous phase channels, one oil phase channel, one junction, and one outlet channel.

17. The method according to claim 15, wherein the cancer is selected from the group consisting of squamous cell carcinoma, small cell lung cancer, non-small cell lung cancer, lung cancer, peritoneal cancer, colon cancer, bile duct tumor, nasopharyngeal cancer, laryngeal cancer, bronchial cancer, thyroid cancer, oral cancer, osteosarcoma, gallbladder cancer, bile duct cancer, kidney cancer, bladder cancer, renal cell cancer, melanoma, brain cancer, glioma, glioblastoma, brain tumor, skin cancer, pancreatic cancer, breast cancer, liver cancer, bone marrow cancer, small intestine cancer, esophageal cancer, large intestine cancer, stomach cancer, eye cancer, urethral cancer, cervical cancer, prostate cancer, ovarian cancer, metastatic cancer, head and neck cancer, rectal cancer, non-Hodgkin's lymphoma, multiple myeloma, acute myelogenous leukemia, lymphoma, acute lymphoblastic leukemia, and chronic myelogenous leukemia.

FIG. 1

FIG. 2

Computer

Aqueous phase
pump (MB)

Oil phase pump
(lipid)

MHF chip

Chip stand

Digital
Microscope

FIG. 3

EP 4 636 402 A1

21

FIG. 4

FIG. 5

FIG. 6

digital microscope

chip stand

Droplet generation chip

Sample reservoir

Droplet outlet

sample pump #1

oil pump

sample pump #2

computer

Droplet Reservoir

NA 0.55
WD.26 mm

FIG. 7

FIG. 8

FIG. 9

FIG. 10

FIG. 11

FIG. 12

a

b

FIG. 13

a

b

FIG. 14

a  Microfluidic cascade

b

c

d  H1975 EVs-spiked in PBS

e  H1975 EVs-spiked in PBS

f  Plasma

FIG. 15

FIG. 16

**Image Acquisition**
Raw images taken using fluorescence microscope
(brightfield, green, red fluorescence)

**Image Breakdown**
Image broken down to brightfield (gray) layer and
fluorescence (green, red) layer

**Brightfield Processing**
Brightfield layer was processed for
droplet quantification

**Fluorescent Processing**
Fluorescent layer was processed to
quantify the number of positive
droplets

**Quantification & Analysis**
Quantification process was done to obtain the
proportion of positive signal among the total
population

FIG. 17

## INTERNATIONAL SEARCH REPORT

| International application No. |
| --- |
| **PCT/KR2023/020308** |

| A. | CLASSIFICATION OF SUBJECT MATTER |
| --- | --- |

**G01N 33/574**(2006.01)i; **C12Q 1/6886**(2018.01)i

According to International Patent Classification (IPC) or to both national classification and IPC

| B. | FIELDS SEARCHED |
| --- | --- |

Minimum documentation searched (classification system followed by classification symbols)

G01N 33/574(2006.01); C12Q 1/6837(2018.01); C12Q 1/6886(2018.01); G01N 21/64(2006.01); G01N 33/543(2006.01); G01N 33/545(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 리포좀(liposomes), 세포외소포체(extracellular vesicles; EV), 종양세포(tumor cells), 검출(detection), 분자비컨(molecular beacons), 캡슐화(encapsulation), 미세유체 액적 반응기(microfluidic droplet reactor), 진단(diagnosis)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X<br><br>A | HU, J. et al. Overhang molecular beacons encapsulated in tethered cationic lipoplex nanoparticles for detection of single-point mutation in extracellular vesicle-associated RNAs. Biomaterials. 2018, vol. 183, pp. 20-29.<br>See abstract; pages 20-27; and figures 1 and 4-6. | 1-8,11-15,17<br><br>9,10,16 |
| A | US 2022-0074929 A1 (THE HONG KONG UNIVERSITY OF SCIENCE AND TECHNOLOGY) 10 March 2022 (2022-03-10)<br>See abstract; and claims 1-20. | 1-17 |
| A | YANG, Y. et al. Ultrafast detection of exosomal RNAs via cationic lipoplex nanoparticles in a micromixer biochip for cancer diagnosis. ACS Applied Nano Materials. 2021, vol. 4, no. 3, pp. 2806-2819.<br>See abstract; pages 2806-2817; and figure 1. | 1-17 |
| A | HU, J. et al. A signal-amplifiable biochip quantifies extracellular vesicle-associated RNAs for early cancer detection. Nature Communications. 2017, vol. 8, article no. 1683, inner pp. 1-11.<br>See abstract; and inner pages 1-8. | 1-17 |

☑ Further documents are listed in the continuation of Box C.    ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
| --- | --- |
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **14 March 2024** | **15 March 2024** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.

**PCT/KR2023/020308**

### C. DOCUMENTS CONSIDERED TO BE RELEVANT

| Category* | Citation of document, with indication, where appropriate, of the relevant passages | Relevant to claim No. |
|---|---|---|
| A | CN 112048554 A (ZHEJIANG UNIVERSITY) 08 December 2020 (2020-12-08)<br>See abstract; and claims 1-6. | 1-17 |
| A | KR 10-2022-0069858 A (INJE UNIVERSITY INDUSTRY-ACADEMIC COOPERATION FOUNDATION) 27 May 2022 (2022-05-27)<br>See abstract; and claims 1-16. | 1-17 |

Form PCT/ISA/210 (second sheet) (July 2022)

| **INTERNATIONAL SEARCH REPORT** | International application No. |
| --- | --- |
| | **PCT/KR2023/020308** |

| **Box No. I** | **Nucleotide and/or amino acid sequence(s) (Continuation of item 1.c of the first sheet)** |
| --- | --- |

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

       ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
Information on patent family members

International application No.

**PCT/KR2023/020308**

| Patent document cited in search report | | | Publication date (day/month/year) | Patent family member(s) | | | Publication date (day/month/year) |
|---|---|---|---|---|---|---|---|
| US | 2022-0074929 | A1 | 10 March 2022 | CN | 109490528 | A | 19 March 2019 |
| | | | | CN | 115047182 | A | 13 September 2022 |
| | | | | WO | 2019-068269 | A1 | 11 April 2019 |
| CN | 112048554 | A | 08 December 2020 | None | | | |
| KR | 10-2022-0069858 | A | 27 May 2022 | None | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SULLIVAN, L. B**. *Nature Chemical Biology*, 2017, vol. 13 (9), 924-925
- **TKACH, M.** ; **THERY, C**. *Cell*, 2016, vol. 164 (6), 1226-1232
- **PEINADO, H. et al.** *Nature Medicine*, 2012, vol. 18 (6), 883-891
- **COCKS, A. et al.** *Seminars in Cancer Biology*, 2021, vol. 75, 127-135
- **WHITTLE, K. et al.** *Critical Reviews in Oncology/-Hematology*, 2022, vol. 171, 103603
- **YU, W. et al.** *Ann Oncol*, 2021, vol. 32 (4), 466-477
- **MARAR, C. et al.** *Nature Immunology*, 2021, vol. 22 (5), 560-570
- **GANDHAM, S. et al.** *Trends Biotechnol*, 2020, vol. 38 (10), 1066-1098
- **SHAO, H. et al.** *Chem Rev*, 2018, vol. 118 (4), 1917-1950
- **ERDBRUGGER, U** ; **LANNIGAN, J.** *Cytometry Part A*, 2016, vol. 89 (2), 123-134
- **BORDANABA-FLORIT, G. et al.** *Nature Protocols*, 2021, vol. 16 (7), 3163-3185
- **KOIKE, S** ; **JAHN, R**. *Nature Communications*, 2019, vol. 10 (1), 1608
- **MA, M** ; **BONG, D**. *Accounts of Chemical Research*, 2013, vol. 46 (12), 2988-2997
- **MAZUR, F** ; **CHANDRAWATI, R**. *ChemNanoMat*, 2021, vol. 7 (3), 223-237
- **YANG, Y. et al.** *Advanced Materials*, 2017, vol. 29 (13), 1605604
- **PIFFOUX, M. et al.** *ACS Nano*, 2018, vol. 12 (7), 6830-6842
- **KUMAR, S. et al.** *Nature Catalysis*, 2021, vol. 4 (9), 763-774
- **CHENG, L. et al.** *Biomaterials*, 2021, vol. 275, 120964
- **PERUZZI, J. A. et al.** *Angew Chem Int Ed Engl*, 2019, vol. 58 (51), 18683-18690
- **GAO, X et al.** *Angewandte Chemie International Edition*, 2019, vol. 58 (26), 8719-8723
- **FENG, J et al.** *Analytical Chemistry*, 2023, vol. 95 (19), 7743-7752
- **WEI PAN et al.** *Anal. Chem.*, 2013, vol. 85 (21), 10581-10588
- **XIANG-HONG PENG et al.** *Cancer Res*, 2005, vol. 65 (5), 1909-1917
- **WOO, H.K. et al.** *Acs Nano*, 2017, vol. 11 (2), 1360-1370
- **MURTAS, G**. *Systems and synthetic biology*, 2010, vol. 4 (2), 85-93
- **JAHN, A et al.** *J Am Chem Soc*, 2004, vol. 126 (9), 2674-5
- **STRYER, L.** ; **HAUGLAND, R. P**. *Proc Natl Acad Sci U S A*, 1967, vol. 58 (2), 719-26
- **FRANCOIS-MARTIN, C** ; **PINCET, F**. *Scientific Reports*, 2017, vol. 7 (1), 43860
- **ZHANG, P. et al.** *Angew Chem Int Ed Engl*, 2001, vol. 40 (2), 402-405
- **HU, J et al.** *Biomaterials*, 2018, vol. 183, 20-29
- **KRICHEVSKY, A. M.** ; **GABRIELY, G**. *J Cell Mol Med*, 2009, vol. 13 (1), 39-53
- **ZHAO, B. X. et al.** *Mol Med Rep*, 2015, vol. 11 (4), 2767-74